# EUROPEAN PATENT SPECIFICATION

(11) **EP 1 912 641 B1**
(45) Date of publication and mention of the grant of the patent: **24.07.2019**
(21) Application number: 06773822.9
(22) Date of filing: 21.06.2006
(51) Int. Cl.: A61K 31/415, A61P 17/16, A61P 39/00

(54) **METHODS AND COMPOSITIONS CONTAINING NATURAL FOLATES FOR PROTECTING AGAINST RADIATION DAMAGE**
VERFAHREN UND ZUSAMMENSETZUNGEN MIT NATÜRLICHEN FOLATEN ZUM SCHUTZ VOR STRAHLENSCHÄDEN
METHODES ET COMPOSITIONS CONTENANT DES FOLATES NATURELS POUR PROTECTION CONTRE LES RADIOLESIONS

(30) Priority: 21.06.2005 US 692401 P
(43) Date of publication of application: 23.04.2008
(73) Proprietor: South Alabama Medical Science Foundation, Mobile, AL 36688 (US); Children's Hospital & Research Center at Oakland, Oakland, CA 94609 (US)
(72) Inventor: BAILEY, Steven, W., Mobile, AL 36688 (US); AYLING, June, E., Mobile, AL 36688 (US); OFFER, Tal, 69494 Tel Aviv (IL); LENTON, Kevin, J., Campbell's Bay, Québec J0X 1K0 (CA)
(74) Representative: Schlich, George
(86) International application number: PCT/US2006/024415
(87) International publication number: WO 2007/002356

(56) References cited:
- WO-A-02/03942
- WO-A-97/27764
- US-A- 5 780 445
- US-A- 5 997 915
- TELEGINA T A ET AL: "Resistance of 5,10-methenyltetrahydrofolate to ultraviolet radiation" APPLIED BIOCHEMISTRY AND MICROBIOLOGY, KLUWER ACADEMIC PUBLISHERS-PLENUM PUBLISHERS, NE, vol. 41, no. 3, 1 May 2005 (2005-05-01), pages 275-282, XP019293788 ISSN: 1608-3024

## Description

### FIELD OF THE INVENTION

The present invention relates, generally, to compositions for use in protecting a subject against radiation damage and, more particularly, to compositions that use natural folates to protect a subject against damage caused by ionizing radiation and ultraviolet radiation.

### BACKGROUND OF THE INVENTION

Humans and other animals are subject to constant exposure to radiation from a variety of sources. Many types of radiation, such as ultraviolet, x-rays, and gamma-rays can cause damage at the molecular and cellular level. Visible light and especially the ultraviolet A and B light in sunlight promote photosensitization reactions after absorption by endogenous and exogenous substances which can then cleave and/or oxidize proteins, lipids, and DNA. Ionizing radiation, on the other hand, can promote formation of very reactive solvated electrons and subsequently hydroxyl radicals.

Living organisms can combat the deleterious effects of radiation either by repairing the damage or by removing the reactive species before they can produce any damage. However, the consequences of exposure to radiation can be quite serious.

For example, occupational doses of ionizing radiation may be received by persons whose job involves exposure (or potential exposure) to radiation, for example, in the nuclear power and nuclear weapons industries. Even in the absence of catastrophic events, workers in the nuclear power industry are subject to higher levels of radiation than the general public.

Military personnel, such as those stationed on vessels powered by nuclear reactors and soldiers required to operate in areas contaminated by radioactive fallout, risk similar exposure to ionizing radiation. Occupational exposure may also occur in rescue and emergency personnel called in to deal with catastrophic events involving a nuclear reactor or radioactive material.

Exposure to ionizing radiation may also result from nuclear weapons detonations (either experimental, as a result of a war, and/or as a result of terrorist activities); from discharges of actinides from nuclear waste storage facilities and nuclear fuel processing and reprocessing centers; from the detonation of so-called "dirty bombs"; from naturally occurring radioactive materials, such as radon gas or uranium; from radiotherapy; from diagnostic x-rays; from cosmic rays and from other exposures to ionizing radiation due to high altitude flight and/or space travel; etc.

A chronic dose is a low level (i.e., 100-5000 millirem) incremental or continuous radiation dose received over time. Examples of chronic doses include a whole body dose of about 5000 millirem per year, which is the dose typically received by an adult working at a nuclear power plant. By contrast, it is recommended that members of the general public should not receive more than 100 millirem per year. Chronic doses may cause long-term cytotoxic and genetic effects, for example, manifesting as an increased risk of a radiation-induced cancer developing later in life. Epidemiologic studies have found that the estimated lifetime risk of dying from cancer is increased by about 0.04% per rem of radiation dose to the whole body.

While anti-radiation suits or other protective gear may be effective at reducing radiation exposure, such gear is expensive, unwieldy, and generally not available to public. Moreover, the use of anti-radiation suits is impractical and/or ineffective against incremental or continuous radiation doses. Therefore, it would be desirable to provide systemic protection from anticipated or inadvertent exposures to ionizing radiation, such as may occur with occupational or environmental exposures.

For all of the above reasons, a need continues to exist for methods and compositions for protecting against radiation damage, and the present invention is directed to addressing this need.

WO0203942(Planet Biotech Inc.) discloses B complex vitamin compositions that protect against cellular damage caused by ultraviolet light. WO0203942 contains anecdotal reports on the effect of vitamin B12 tablets, or vitamin B12 in combination with folic acid, on susceptibility to sunburn.

WO9727764 (Univ. South Alabama)discloses food and vitamin preparations containing the natural isomers of reduced folates.

US5997915 (Steven W. Bailey)discloses compositions for human and animal consumption containing reduced folates.

Sonka et al., Experimental Radiobiology, 10, 101-1-3, 1966, and Okazaki et al., Chem. Pharm. Bull., 19(6), 1173-1177, 1971, each describe experiments designed to investigate the therapeutic effect of tetrahydrofolic acid in mice that had been irradiated with X-rays.

Schunzel, Acta Histochemica, Suppl. Band XXX, S, 341-345, 1984, Schünzel et al., Radiobiol.-Radiother., Vol 16(2), 173-180, 1975, and Schünzel et al., Radiobiol.-Radiother., Vol 10(5), 685-7, 1969, each describe experiments in which test animals that had been subjected to X-ray irradiation were treated by intraperitoneal injection of either L-cysteine or a mixture of L-cysteine and tetrahydrofolic acid.

Keshava et al., Mutation Research, 352 (1996), 123-134, describes studies on the inhibitory effect of folinic acid on radiation-induced micronuclei and chromosomal aberrations in V79 cells.

### SUMMARY OF THE INVENTION

The present invention, in one aspect thereof, relates to a reduced folate for use in a method of protecting a human subject from harmful effects of ionizing radiation, said method comprising administering to the subject an effective amount of at least one said reduced folate; wherein the reduced folate is selected from the group consisting of 5-methyl-tetrahydrofolic acid, and polyglutamyl derivatives thereof.

The present invention also relates to a radioprotective composition for use in providing radioprotection against ionising radiation to a human subject, the composition comprising: a first radioprotective agent, said first radioprotective agent being a reduced folate; and a second radioprotective agent, wherein the reduced folate is selected from the group consisting of 5-methyl-tetrahydrofolic acid, and polyglutamyl derivatives thereof; wherein the second radioprotective agent is selected from an iodide salt and a steroidal radioprotective agent.

The present invention also relates to a reduced folate for use in a method for protecting a human subject from harmful effects of ultraviolet radiation, said method comprising: administering to the subject a composition comprising an effective amount of at least one said reduced folate, wherein said composition is substantially free from vitamin B12 in that it contains no vitamin B12 or contains an amount of vitamin B12 that is equal to or less than the recommended daily allowance of vitamin B12 for a human subject and said recommended daily allowance is 6 pg/day, wherein the reduced folate is selected from the group consisting of 5-methyl-tetrahydrofolic acid, and polyglutamyl derivatives thereof, and monoalkyl, dialkyl, monobenzyl and/or dibenzyl esters of a glutamate side chain of the reduced folate.

### BRIEF DESCRIPTION OF THE DRAWINGS

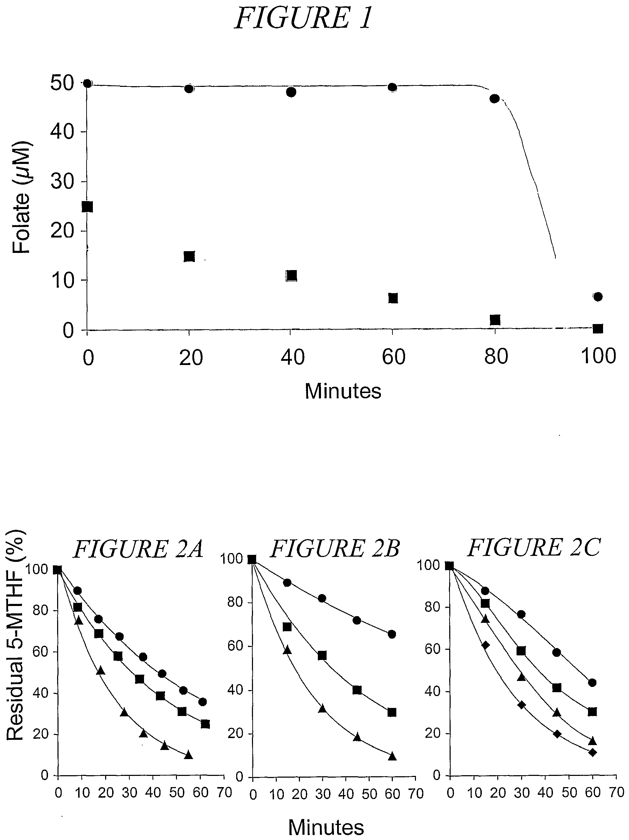

Figure 1 is a graph showing the inhibition of folic acid photodegradation by 5-methyltetrahydrofolate ("5-MTHF"). Samples were taken during light exposure at various times and analyzed by HPLC on a Luna phenyl-hexyl 5 :m (25 x 0.46 cm) column (Phenomenex) eluted at a flow rate of 1.5 ml/min with ammonium phosphate (20 mM in ammonium), pH 2.8 / acetonitrile (17:1) with detection by UV absorbance using Waters 996, photodiode array spectrometer. Squares indicate 5-MTHF concentration, and circles indicate folic acid concentration.
Figures 2A-2C are graphs showing the photodegradation of 5-MTHF by Rose Bengal. In Figure 2A, 5-MTHF, at 25 :M, pH 7.4, was illuminated by light from a 40 W tungsten lamp passed through a Wratten #16 gelatin filter in the presence of 5 :M Rose Bengal and 900 U of superoxide dismutase ("SOD") in 100 % O₂ (circles), air (squares), or 1.8 % O₂ in argon (triangles). In Figure 2B, 5-MTHF, at 25 :M, pH 7.4, was illuminated by light from a 40 W tungsten lamp passed through a Wratten #16 gelatin filter in the presence of 100 % O₂ and in the presence of 10 :M Rose Bengal and SOD with: no azide (triangles), 0.5 mM azide (squares), or 5 mM azide (circles). In Figure 2C, 5-MTHF, at 25 :M, pH 7.4, was illuminated by light from a 40 W tungsten lamp passed through a Wratten #16 gelatin filter in the presence of 100 % O₂ and in the presence of 10 :M Rose Bengal and SOD with: no ascorbate (diamonds), 0.2 mM ascorbate (triangles), 1 mM ascorbate (squares), or 2 mM ascorbate (circles).

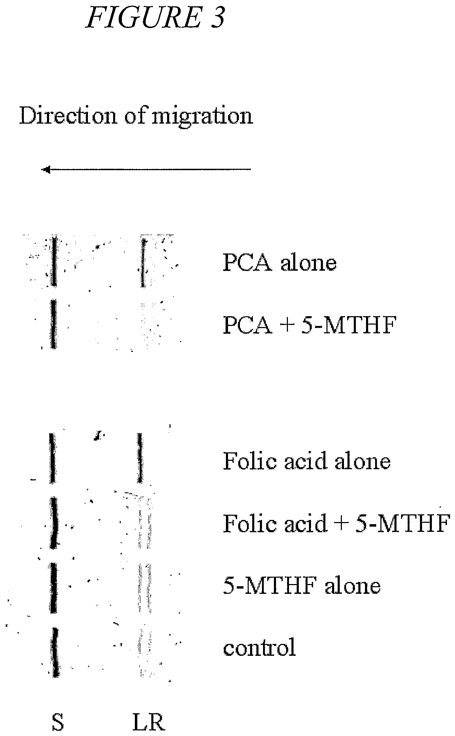

Figure 3 is an image of gel electrophoresis experiments showing that 5-MTHF inhibits UVA-mediated DNA damage catalyzed by pterin-6 carboxylic acid ("PCA"). In the top panel, supercoiled plasmid DNA, PBR 322 (0.1 :g) was exposed for 80 min to UVA in the presence of 50 :M of PCA alone or in the presence of PCA together with continuous addition of 5-MTHF as described in Example 4. In the bottom panel, supercoiled plasmid DNA, PBR 322 (0.1 :g) was exposed for 80 min to UVA in the presence of 50 :M of folic acid alone, folic acid added together with 50 :M 5-MTHF, or 5-MTHF alone. In each case, the reaction mixture (10 :1) was then subjected to agarose gel electrophoresis. Positions of a supercoiled form (S), a nicked circular form (relaxed) (R), and a linear form (L) are indicated.

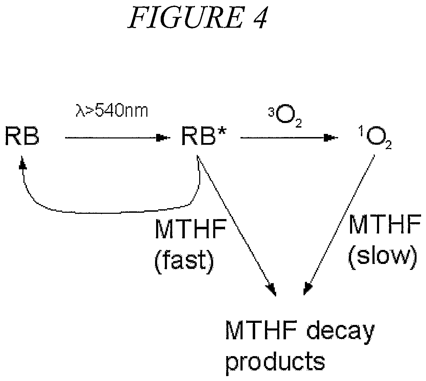

Figure 4 sets forth a possible mechanism of 5-MTHF photo-antioxidative activity. 5-MTHF is depleted at a faster rate in low oxygen when singlet oxygen concentration is low due to directly reacting with photoactivated Rose Bengal ("RB"). At high O₂ levels, which lead to increased formation of singlet oxygen, the depletion of 5-MTHF slows.

### DETAILED DESCRIPTION OF THE INVENTION

The present invention, in one aspect thereof, relates to a reduced folate for use in a method of protecting a human subject from harmful effects of ionizing radiation, said method comprising administering to the subject an effective amount of at least one said reduced folate; wherein the reduced folate is selected from the group consisting of 5-methyl-tetrahydrofolic acid, and polyglutamyl derivatives thereof.

"Subject", as used herein, is meant to refer to any organism that would benefit from protection from one or more harmful effects of ionizing radiation. Examples of suitable subjects include animals, such as mammals, domestic animals, wild animals, bovine animals, equine animals, porcine animals, canine animals, feline animals, murine animals, goats, cows, cattle, sheep, pigs, horses, dogs, cats, rabbits, mice, rats, tigers, bears, lions, birds, marsupials, and the like. "Subject", as used herein, is also meant to include humans, such as male humans, female humans, adult humans, adolescent humans, and children. By way of illustration, suitable subjects are meant to include those humans or other subjects who are incurring exposure to harmful levels or potentially harmful levels of ionizing radiation; as well as those humans or other subjects who are at risk for incurring exposure to harmful levels or potentially harmful levels of ionizing radiation. In this context, "harmful level of ionizing radiation" is meant to refer to any level of ionizing radiation that is greater than mere background levels. "Harmful level of ionizing radiation" is meant to include acute radiation doses of more than about 1000 millirem, such as more than about 2000 millirem, more than about 3000 millirem, more than about 4000 millirem, more than about 5000 millirem, more than about 10,000 millirem, more than about 20,000 millirem, more than about 50,000 millirem, more than about 100,000 millirem, more than about 200,000 millirem, and/or more than about 500,000 millirem. "Harmful level of ionizing radiation" is meant to also include continuous, intermittent, or other forms of chronic radiation doses totaling more than about 100 millirem per year, such as more than about 200 millirem per year, more than about 300 millirem per year, more than about 400 millirem per year, more than about 500 millirem per year, more than about 600 millirem per year, more than about 700 millirem per year, more than about 800 millirem per year, more than about 900 millirem per year, more than about 1000 millirem per year, more than about 2000 millirem per year, more than about 3000 millirem per year, more than about 4000 millirem per year, more than about 5000 millirem per year, and/or more than about 10,000 millirem per year. Suitable human subjects include those who are employed at or visiting a nuclear power facility (e.g., nuclear power plants, nuclear fuel processing or reprocessing facilities, nuclear fuel storage facilities, etc.); those who live, work, attend school, or otherwise spend a significant amount of time near a nuclear power facility (e.g., nuclear power plants, nuclear fuel processing or reprocessing facilities, nuclear fuel storage facilities, etc.); those who are stationed on or visiting nuclear powered submarines and other kinds of nuclear powered marine vessels; those who are stationed on or visiting nuclear powered submarines and other kinds of nuclear powered marine vessels; civilians living or operating in areas contaminated by nuclear weapons fallout; military personnel operating in areas contaminated by nuclear weapons fallout; emergency personnel who deal with nuclear accidents; civilians, military personnel, and emergency personnel living or operating in areas contaminated by release of radioactive materials by terrorists; those who live, work, attend school, or otherwise spend a significant amount of time in structures having high levels of radon gas; astronauts and other space travelers; those who frequently fly at high altitude, for example, pilots, airline attendants, etc.); those who suffer from defects in nucleic acid repair enzymes; etc. Additionally or alternatively, the subject can be one who is folate deficient, or the subject can be one who is not folate deficient. As used herein, a subject is to be viewed as being folate deficient if the subject's homeostatic plasma level of reduced folate is below the norm for that subject. In the case of human subjects, a human subject is to be viewed, for the purposes of the present invention, as being folate deficient if the human subject's homeostatic plasma level of reduced folate is below 20 nanomolar. Conversely, for the purposes of the present invention, a human subject is to be viewed as not being folate deficient if the human subject's homeostatic plasma level of reduced folate is at or above 20 nanomolar.

"Ionizing radiation", as used herein, is meant to include, for example, x-rays, gamma rays, cosmic rays, beta particles, alpha particles, high-energy heavier nuclei, high-energy protons, fast electrons, positrons, and solar particles. The exposure to ionizing radiation can be the result of a variety of activities, such as exposures due to high altitude flight, space travel, radiation therapy, accidents, and the like.

"Protecting", as used in the context of ionizing radiation, is meant to refer to any measurable or otherwise observable reduction in one or more of the harmful effects of ionizing radiation. Such reduction in a harmful effect can be ascertained directly, e.g., by monitoring DNA or other cellular changes, or indirectly, by qualitatively or quantitatively evaluating a subject's symptoms resulting from ionizing radiation exposure. As indicated above, the protection need not be and, in many cases, will not be a complete (100%) reduction in the harmful effects of ionizing radiation. For the purposes of illustration, any reduction in any one (or two or three or more) of the harmful effects of ionizing radiation is to be construed as "protecting" the subject from harmful effects of ionizing radiation. Such reduction can be observed in terms of the severity of the harmful effect, the duration of the harmful effect, or both; and, as mentioned above, it can be qualitative or quantitative.

Examples of harmful effects of ionizing radiation from which a subject can be protected in accordance with the present invention include: radiation sickness, hair loss (alopecia), weakness, fatigue, nausea, vomiting, diarrhea, skin burns, gastrointestinal tract bleeding, mucous membrane bleeding, gastrointestinal sloughing, oral mucosal sloughing, genetic defects, hematopoietic and/or immunocompetent cell destruction, sterility, bone marrow cancer and other kinds of cancer, premature aging, death, venoocclusive disease of the liver, chronic vascular hyperplasia of cerebral vessels, cataracts, and pneumonites.

The reduced folate can be administered prior to and/or during the subject's exposure to ionizing radiation, depending (in part) on the nature of the ionizing radiation exposure. For example, where exposure is chronic (or where the risk of exposure is elevated over a long period of time) the reduced folates can be administered on a regular basis, for example, once per day, multiple times per day (e.g., twice per day, thrice per day, four times per day, six times per day, etc.), or continuously (e.g., as in the case where the reduced folate is administered in a time-release formulation). The reduced folates can be administered so as to maintain plasma concentrations above homeostatic levels for the period of time during which protection is desired. For the purposes of the present invention, the homeostatic level is the concentration of reduced folate in the plasma from blood, as measured while fasting and after about 24 hours of any prior folate supplementation. Plasma levels need not be determined for each individual, but, rather, they can be projected on the basis of pharmacokinetic data from a group of subjects.

It is believed that the protective effects of reduced folates become optimal at a time after their concentration in the plasma reaches a maximum. The time for this maximum concentration to occur (Tmax) can depend on the formulation in which the reduced folate is administered and the dose. For example, a solution formulation achieves a Tmax typically between 0.5 and 2.0 hours (e.g., between 0.5 and 1.0 hours), whereas other formulations can have longer Tmax. Illustratively, where a radiation exposure is anticipated to occur at a known future time, it is desirable to administer (or commence administration of) reduced folate at about Tmax (0.5 and 2 hours for a solution formulation) prior to the anticipated time of the radiation exposure. Earlier administration (i.e., more than Tmax prior to the anticipated time of the radiation exposure) or later administration (i.e., less than Tmax prior to the anticipated time of the radiation exposure) still results in some level of protection, although this the level of protection may not be optimal. As indicated above and as discussed further below, it is advantageous to commence administration at least Tmax prior to the anticipated time of the radiation exposure and to continue regular administration of reduced folate (e.g., one or more times per day) for the period of time during which the subject is exposed to ionizing radiation. Multiple consecutive doses or a time release formulation can be used to lengthen the time during which plasma levels of reduced folate are in excess of homeostatic levels. Intravenous administration can be used to achieve a quicker increase in plasma concentrations of reduced folate. In the case of human subjects, reduced folate can be administered so as to attain and/or maintain the subject's plasma level of reduced folate at a value greater than 20 nanomolar, such as greater than about 30 nanomolar, greater than 40 nanomolar, greater than about 50 nanomolar, greater than 60 nanomolar, greater than about 70 nanomolar, greater than about 80 nanomolar, greater than about 90 nanomolar, greater than about 100 nanomolar, greater than about 150 nanomolar, greater than about 200 nanomolar, greater than about 250 nanomolar, greater than about 300 nanomolar, greater than about 350 nanomolar, greater than about 400 nanomolar, greater than about 450 nanomolar, greater than about 500 nanomolar, greater than about 600 nanomolar, greater than about 700 nanomolar, greater than about 800 nanomolar, greater than about 900 nanomolar, greater than about 1 micromolar, greater than about 2 micromolar, greater than about 5 micromolar, greater than about 10 micromolar, greater than about 20 micromolar, greater than about 30 micromolar, greater than about 40 micromolar, greater than about 50 micromolar, etc.

In another embodiment of the present invention, reduced folate is administered routinely (e.g., daily) to the subject so that the subject's homeostatic plasma level of reduced folate is elevated to a value above that at which the subject would be considered to be folate deficient. For example, in the case of human subjects, reduced folate can be administered routinely (e.g., daily) to the human subject so as to increase the human subject's homeostatic plasma level of reduced folate to a value greater than 20 nanomolar, such as greater than about 30 nanomolar, greater than 40 nanomolar, greater than about 50 nanomolar, greater than 60 nanomolar, greater than about 70 nanomolar, greater than about 80 nanomolar, greater than about 90 nanomolar, greater than about 100 nanomolar, greater than about 120 nanomolar, greater than about 140 nanomolar, greater than about 160 nanomolar, greater than about 180 nanomolar, greater than about 200 nanomolar, etc. By increasing the subject's homeostatic plasma level of reduced folate to a level that is higher than has been considered in the art to be sufficient, the method of the present invention can be used to protect the subject from unanticipated exposures to ionizing radiation.

As discussed above, the reduced folate can be administered prior to and/or during the subject's exposure to ionizing radiation, depending (in part) on the nature of the ionizing radiation exposure. It will be appreciated that a subject who has been exposed to ionizing radiation involving radioactive materials may have become contaminated with radioactive materials (e.g., by inhalation of radioactive gasses, by ingestion of radioactive matter, by contamination of skin or clothes, by absorption of radioactive iodine, etc.), and, therefore, the subject may continue to be exposed to ionizing radiation (or may be at risk for continued exposure to ionizing radiation) for a period of time after the subject leaves or is removed from the primary source of ionizing radiation (e.g., the site of a nuclear accident, the site of a nuclear attack, the site of a terrorist's radiologic/nuclear detonation, etc.). Administration of reduced folate after the subject leaves or is removed from the primary source of ionizing radiation but while the subject continues to be exposed to ionizing radiation as a result of having become contaminated with radioactive materials (or while the subject is at risk for such a continued exposure) is to be viewed as being an administration "during" the subject's exposure to ionizing radiation.

As discussed above, the present invention involves administering at least one reduced folate to the subject wherein the reduced folate is selected from the group consisting of 5-methyl-tetrahydrofolic acid, and polyglutamyl derivatives thereof. These can be administered as their natural isomers: 5-methyl-(6S)-tetrahydrofolic acid, and polyglutamyl derivatives thereof. The aforementioned natural isomers can be administered in combination with a corresponding non-natural isomer 5-methyl-(6R)-tetrahydrofolic acid and polyglutamyl derivatives thereof), or they can be administered alone (i.e., substantially free from the corresponding non-natural isomer). Illustratively, suitable reduced folates include racemic 5-methyl-tetrahydrofolic acid and polyglutamyl derivatives thereof.

The term "Reduced folate" is also meant to include polyglutamyl derivatives; as well as monoalkyl, dialkyl, monobenzyl, and/or dibenzyl esters of the reduced folate's glutamate side chain. It is believed that monoalkyl, dialkyl, monobenzyl, and/or dibenzyl esters of the reduced folate's glutamate side chain are especially useful in topical formulations.

The reduced folates can be in either in the form of a free acid or in the form of a salt, and "reduced folate", as used herein, is also meant to encompass both the free acid and salt forms. Examples of suitable salt forms include hydrochloride, sodium, potassium, and magnesium salts. As yet another example, the reduced folate can be in the form of a calcium salt. The salt form and crystal structure of the reduced folate somewhat affects the reduced folate's stability and solubility, and this can be optimized depending on the needs for a particular formulation. Suitable salt forms also include those in which the counter ion is an organic amine base. The pH of the final composition can also be optimized according to the stability properties of the particular reduced folate used and the other components present in the formulation (if any), as is well understood in the arts of nutrient processing and folate compounds.

The reduced folate can be administered alone or in a composition containing, in addition to the reduced folate, one or more other components. Examples of suitable dosage forms include enteral (e.g., oral, intragastric, or transpyloric), parenteral (intramuscular, intravenous, intraperitoneal, rectal, vaginal, and subcutaneous), topical, and ocular dosage forms.

Illustratively, the reduced folate can be administered orally in the form of a supplement. For example, pills, tablets, chewable tablets, capsules, powders, syrups, suspensions, solutions, and soft chews are suitable forms for administration of reduced folates for protection against ionizing radiation. Time delay, slow-release, and enterically-protected formulations can also be used. Suitable dosage forms for orally administered supplements include tablets, dispersible powders, granules, capsules, suspensions, syrups, and elixirs. Inert diluents and carriers for tablets include, for example, calcium carbonate, sodium carbonate, lactose, and talc. Tablets may also contain granulating and disintegrating agents, such as starch and alginic acid; binding agents, such as starch, gelatin, and acacia; and lubricating agents, such as magnesium stearate, stearic acid, and talc. Tablets may be uncoated or may be coated by known techniques to delay disintegration and absorption. Inert diluents and carriers which may be used in capsules include, for example, calcium carbonate, calcium phosphate, and kaolin. Suspensions, syrups, and elixirs may contain conventional excipients, for example, methyl cellulose, tragacanth, sodium alginate; wetting agents, such as lecithin and polyoxyethylene stearate; and preservatives, such as ethyl-p-hydroxybenzoate. Other inert ingredients can also be present in the dosage forms for oral administration.

As discussed above, dosage forms for oral administration can include inert materials, such as fillers, binding agents, stabilizers, sweeteners, including nutritive sweeteners (e.g. sucrose, sorbitol, and other polyols) and non-nutritive sweeteners (e.g. saccharin, aspartame, and acesulfame K), colorants, flavors, buffers, salts, coatings, and the like that are known to those skilled in the art of supplement and pharmaceutical formulation. Additionally or alternatively, the oral dosage forms can also include one or more additional (i.e., in addition to the reduced folate) biologically active materials. Examples of such additional biologically active materials that can be present in the composition include: other vitamins and/or nutrients (e.g., folic acid; vitamin B1; vitamin B2; vitamin B3; vitamin B5; vitamin B6; vitamin B12; vitamin C; vitamin A and its precursors, such as beta-carotene; vitamin D; vitamin E including vitamin E isomeric forms and derivatives; vitamin K; biotin; pantothenic acid; methionine; choline; taurine; carnitine; acetyl-carnitine; sugars; lipids; amino acids, such as glutamine, arginine, and methionine; and proteins), reducing agents and antioxidants, radioprotective agents (e.g., iodides, such as potassium iodide and other iodide salts; steroidal radioprotective agents, especially steroids and steroid derivatives know to be useful for enhancing the protective response of the immune system, for example, DHEA, 5-androstenediol and other androstenediols and androstenetriol and their derivatives), thiols (e.g., glutathione and glutathione elevating precursors, glutamine, cysteine, N-acetyl-cysteine, alpha-lipoic acid, cystinyl-glycine, cyctamine, S-allyl cysteine sulfoxide, aminoethylisothiourea, mercaptoethyl guanidine, 2-mercaptopropionylglycine), selenium salts, selenized yeast, selenomethioine, Co-enzyme Q10, amifostine, N-t-butyl hydroxylamine and other N-hydroxylamine derivatives, melatonin, superoxide dismutase, its derivatives and mimetic-metal complexes, chelating agents, phytochemicals, polyphenols, extracts of natural products including herbs, chinese herbs, ayurvedic preparations, tea extracts, dithiolthiones, cruciferous vegetables, flavanoids, curcumin, methylxanthines, Gingko biloba extracts, and minerals (e.g., boron, calcium, phosphorus, chromium, copper, manganese, magnesium, nickel, sodium, molybdenum, potassium, iron, selenium, silicon, vanadium, and zinc). As further illustration, the additional biologically active materials that can be used in the compositions of the present invention include essential nutrients, such as those that have been compiled in a number of published sources, including Modern Nutrition in Health and Disease, 8th ed., Shils et al., eds., Philadelphia:Lea and Febiger (1994).

By way of illustration, in one embodiment of the present invention, the reduced folate is administered in a composition that includes the reduced folate and one or more radioprotective agents. Examples of radioprotective agents include an iodide salt, such as potassium iodide present in an amount effective to reduce absorption of radioiodines from the environment. Although lower levels of iodide supplementation can offer some degree of protection against radioiodine uptake by the thyroid, daily doses of from about 1 mg to about 500 mg (e.g., from about 8 mg to about 260 mg of KI per day, from about 16 mg to about 130 mg) are believed to be particularly effective. Details regarding the use of KI to reduce absorption of radioiodines from the environment are presented, for example, in "Guidance: Potassium Iodide as a Thyroid Blocking Agent in Radiation Emergencies," Rockville, Maryland: U.S. Department of Health and Human Services, Food and Drug Administration, Center for Drug Evaluation and Research (CDER) (December 2001). As further illustration, the radioprotective agent can be a steroidal radioprotective agent, such as an androstenediol. As one skilled in the art will appreciate, the composition that includes the reduced folate and additional radioprotective agent (i.e., in addition to the radioprotective reduced folate) can further include other components, such as inert materials and/or biologically active materials, such as in the case where the composition further includes, in addition to the reduced folate, one or more other vitamins. Illustratively, such compositions are substantially free from vitamin B12. As used in this context, "substantially free from vitamin B12" is meant to refer to compositions in which the level of vitamin B12 present in the composition is insufficient to have an appreciable effect on the protection from harmful effects of ionizing radiation that the composition affords. Illustratively, in the context of compositions for protecting a subject from harmful effects of ionizing radiation, compositions containing no vitamin B12; containing vitamin B12 in an amount that is equal to or less than the recommended daily allowance of vitamin B12 for the subject. As used herein, "recommended daily allowance" is meant to refer to the recommended daily allowance in the United States, which, for vitamin B12, is 6 :g/day.

The reduced folate can also be administered orally as a food that is fortified with one or more reduced folates. Foods can be single-component foods, for example, fruits and fruit juices (e.g., orange juice), dairy products (e.g., milk), vegetables (e.g., spinach), other such single-component foods. Foods can also be multi-component preparations made from two or more single-component foods. Typically, foods contain various concentrations of endogenous reduced folates. Depending on the nature of the processing needed, the fortification is often optimally performed after any especially destructive processing steps, as is well know in the art of food fortification. Since the amount endogenous reduced folates present in the food can vary, it can be advantageous to know the final amount (number of moles) of reduced folate in the food or food preparation, as quantified, for example, by analysis of a sample of a product batch. Many analytical methods (such as microbial growth dependence, folate binding protein based assays, HPLC and GC) are available for measurement of the reduced folate content of foods, food preparations, and supplements.

Irrespective of whether the reduced folate is administered orally to a human subject in the form of a supplement, in the form of a fortified food, or in the form of a food preparation, the total amount of reduced folate administered per dose can be in the range of from about 0.45 micromoles to about 2 millimoles (based on the natural isomer component, if the reduced folate is present as a mixture of isomers), such as from 0.45 micromoles to 2 millimoles, from about 0.9 micromoles to about 2 millimoles, from 0.9 micromoles to 2 millimoles, from about 1.8 micromoles to about 2 millimoles, from 1.8 micromoles to 2 millimoles, from about 0.45 micromoles to about 1 millimole, from about 0.9 micromoles to about 1 millimole, from about 1.8 micromoles to about 1 millimole, from about 0.45 micromoles to about 0.5 millimoles, from about 0.9 micromoles to about 0.5 millimoles, from about 1.8 micromoles to about 0.5 millimoles, from about 0.45 micromoles to about 100 micromoles, from about 0.9 micromoles to about 100 micromoles, and/or from about 1.8 micromoles to about 100 micromoles.

As discussed above, oral administration can be carried out in a single dose, multiple doses, or continuously. The amount of reduced folate contained in a single dose will, of course, depend in part on the dosing regimen and the total amount of reduced folate to be administered to the subject in a given period of time (e.g., per day). Suitable daily dosage ranges for protection against chronic exposure to ionizing radiation include: from about 0.45 micromoles to about 15 micromoles, from about 0.9 micromoles to about 15 micromoles, from 0.9 micromoles to 15 micromoles, from about 1 micromoles to about 15 micromoles, from about 2 micromoles to about 12 micromoles, from about 3 micromoles to about 10 micromoles, from about 5 micromoles to about 8 micromoles, etc.

Dosage forms suitable for parenteral administration include solutions, suspensions, dispersions, emulsions, and the like. They may also be manufactured in the form of sterile solid compositions which can be dissolved or suspended in sterile injectable medium immediately before use. They may contain suspending or dispersing agents known in the art. Examples of parenteral administration are intramuscular, intravenous, rectal, and subcutaneous administration.

As mentioned above, the reduced folates can be administered via routes other than oral and parenteral routes. For example, the reduced folates can be administered to the eye in the form of drops, creams, or gel solutions or suspensions adapted for ocular application. The reduced folates can also be administered topically, for example in a conventional topical cream, lotion, spray, or gel matrix. Topical formulations can benefit from incorporation of delivery systems that enhance skin penetration (e.g., liposomes, etc.), as is known in the art. Illustratively, topical dosage forms can be formulated so as to contain from about 0.05 micromoles to about 1 millimole, such as from 0.05 micromoles to 1 millimole, from about 0.1 micromoles to about 0.5 millimole, from about 0.5 micromoles to about 0.1 millimole (based on the natural isomer component, if the reduced folate is present as a mixture of isomers) per square meter of coverage area. It is preferred that topical formulations be applied, in advance, to the subject's skin in areas of anticipated exposure. Additional applications may be useful to maintain the presence of the reduced folates over a long period of time, or additional applications may be useful in the event of significant exposure to water.

Further details regarding formulating the compositions described hereinabove, such as compositions for topical, enteral, and parenteral administration, can be found, for example, in Handbook of Pharmaceutical Excipients, 3rd Edition (2000), American Pharmaceutical Association; The Theory and Practice of Industrial Pharmacy, 3rd Edition, Lachman et al. 1986; Pharmaceutical Dosage Forms: Tablets Volume Edition, Christopher T, edition, 1995; and Remington's Pharmaceutical Sciences, 2000.

It will be appreciated that the actual preferred amount of reduced folate to be administered according to the present invention will vary according to the particular reduced folate, the particular composition formulated, and the mode of administration. Many factors that may modify the action of the reduced folate (e.g., body weight, sex, diet, time of administration, route of administration, rate of excretion, condition of the subject, drug combinations, reaction sensitivities and severities, and the type, intensity, and duration of the ionizing radiation to which the subject is exposed) can be taken into account by those skilled in the art. Administration can be carried out continuously or periodically within the maximum tolerated dose. Optimal administration rates for a given set of conditions can be ascertained by those skilled in the art using conventional dosage administration tests.

The present invention, in another aspect thereof, relates to a reduced folate for use in a method for protecting a human subject from harmful effects of ultraviolet radiation, said method comprising: administering to the subject a composition comprising an effective amount of at least one said reduced folate, wherein said composition is substantially free from vitamin B12 in that it contains no vitamin B12 or contains an amount of vitamin B12 that is equal to or less than the recommended daily allowance of vitamin B12 for a human subject and said recommended daily allowance is 6 pg/day, wherein the reduced folate is selected from the group consisting of 5-methyl-tetrahydrofolic acid, and polyglutamyl derivatives thereof, and monoalkyl, dialkyl, monobenzyl and/or dibenzyl esters of a glutamate side chain of the reduced folate.

As discussed above, "subject", as used herein, is meant to refer to any organism that would benefit from protection from one or more harmful effects of ultraviolet radiation. Examples of suitable subjects include animals, such as mammals, domestic animals, wild animals, bovine animals, equine animals, porcine animals, canine animals, feline animals, murine animals, goats, cows, cattle, sheep, pigs, horses, dogs, cats, rabbits, mice, rats, tigers, bears, lions, birds, marsupials, and the like. "Subject", as used herein, is also meant to include humans, such as male humans, female humans, adult humans, adolescent humans, and children. By way of illustration, suitable subjects are meant to include those humans or other subjects who are incurring exposure to harmful levels or potentially harmful levels of ultraviolet radiation; as well as those humans or other subjects who are at risk for incurring exposure to harmful levels or potentially harmful levels of ultraviolet radiation (e.g., individuals who expect to be working or otherwise be outdoors for extended periods of time and individuals who will be exposed to artificial sources of ultraviolet radiation, such as from a tanning bed). Additionally or alternatively, the subject can be one who is folate deficient, or the subject can be one who is not folate deficient. As used herein, a subject is to be viewed as being folate deficient if the subject's homeostatic plasma level of reduced folate is below the norm for that subject. In the case of human subjects, a human subject is to be viewed, for the purposes of the present invention, as being folate deficient if the human subject's homeostatic plasma level of reduced folate is below 20 nanomolar. Conversely, for the purposes of the present invention, a human subject is to be viewed as not being folate deficient if the human subject's homeostatic plasma level of reduced folate is at or above 20 nanomolar.

"Ultraviolet radiation", as used herein, is meant to include, for example, UV-A radiation, UV-B radiation, UV-C radiation, vacuum UV radiation, and combinations of two or more of the above kinds of ultraviolet radiation.

"Protecting", as used in the context of ultraviolet radiation , is meant to refer to any measurable or otherwise observable reduction in one or more of the harmful effects of ultraviolet radiation. Such reduction in a harmful effect can be ascertained directly, e.g., by monitoring DNA or other cellular changes, or indirectly, by qualitatively or quantitatively evaluating a subject's symptoms resulting from exposure to ultraviolet radiation. As indicated above, the protection need not be and, in many cases, will not be a complete (100%) reduction of the harmful effects of ultraviolet radiation. For the purposes of illustration, any reduction in any one (or two or three or more) of the harmful effects of ultraviolet radiation is to be construed as "protecting" the subject from harmful effects of ultraviolet radiation. Such reduction can be observed in terms of the severity of the harmful effect, the duration of the harmful effect, or both; and, as mentioned above, it can be qualitative or quantitative.

Examples of harmful effects of ultraviolet radiation from which a subject can be protected in accordance with the method of the present invention include: photo aging of skin, wrinkling of skin, damage to DNA or other forms of cellular damage, increased risk or incidence of precancerous skin lesions, increased risk or incidence of cancerous lesions, increased risk or incidence of melanomas and other kinds of cancers, and death.

The natural folate can be administered prior to and/or during the subject's exposure to ultraviolet radiation, depending (in part) on the nature of the ultraviolet radiation exposure. For example, where exposure is chronic (or where the risk of exposure is elevated over a long period of time) the reduced folates can be administered on a regular basis, for example, once per day, multiple times per day (e.g., twice per day, thrice per day, four times per day, six times per day, etc.), or continuously (e.g., as in the case where the reduced folate is administered in a time-release formulation). The reduced folates can be administered so as to maintain plasma concentrations above homeostatic levels for the period of time during which protection is desired. As discussed above, for the purposes of the present invention, the homeostatic level is the concentration of reduced folate in the plasma from blood, as measured while fasting and after about 24 hours of any prior folate supplementation. Plasma levels need not be determined for each individual, but, rather, they can be projected on the basis of pharmacokinetic data from a group of subjects.

It is believed that the protective effects of reduced folates become optimal at a time after their concentration in the plasma reaches a maximum. The time for this maximum concentration to occur (Tmax) can depend on the formulation in which the reduced folate is administered and the dose. For example, a solution formulation achieves a Tmax typically between 0.5 and 2.0 hours (e.g., between 0.5 and 1.0 hours), whereas other formulations can have longer Tmax. Illustratively, where an exposure to ultraviolet radiation is anticipated to occur at a known future time, it is desirable to administer (or commence administration of) reduced folate at about Tmax (0.5 and 2 hours for a solution formulation) prior to the anticipated time of the radiation exposure. Earlier administration (i.e., more than Tmax prior to the anticipated time of the ultraviolet exposure) or later administration (i.e., less than Tmax prior to the anticipated time of the ultraviolet exposure) still results in some level of protection, although this the level of protection may not be optimal. As indicated above and as discussed further below, it is advantageous to commence administration at least Tmax prior to the anticipated time of ultraviolet exposure and to continue regular administration of reduced folate (e.g., one or more times per day) for the period of time during which the subject is exposed to ultraviolet radiation. Multiple consecutive doses or a time release formulation can be used to lengthen the time during which plasma levels of reduced folate are in excess of homeostatic levels. As still further illustration, in the case of human subjects, reduced folate can be administered to the human subject so as to attain and/or maintain the subject's plasma level of reduced folate at a value greater than 20 nanomolar, such as greater than about 30 nanomolar, greater than 40 nanomolar, greater than about 50 nanomolar, greater than 60 nanomolar, greater than about 70 nanomolar, greater than about 80 nanomolar, greater than about 90 nanomolar, greater than about 100 nanomolar, greater than about 150 nanomolar, greater than about 200 nanomolar, greater than about 250 nanomolar, greater than about 300 nanomolar, greater than about 350 nanomolar, greater than about 400 nanomolar, greater than about 450 nanomolar, greater than about 500 nanomolar, greater than about 600 nanomolar, greater than about 700 nanomolar, greater than about 800 nanomolar, greater than about 900 nanomolar, greater than about 1 micromolar, etc.

In another embodiment of the present invention, reduced folate is administered routinely (e.g., daily) to the subject so that the subject's homeostatic plasma level of reduced folate is elevated to a value above that at which the subject would be considered to be folate deficient. For example, in the case of human subjects, reduced folate can be administered routinely (e.g., daily) to the human subject so as to increase the human subject's homeostatic plasma level of reduced folate to a value greater than 20 nanomolar, such as greater than about 30 nanomolar, greater than 40 nanomolar, greater than about 50 nanomolar, greater than 60 nanomolar, greater than about 70 nanomolar, greater than about 80 nanomolar, greater than about 90 nanomolar, greater than about 100 nanomolar, greater than about 120 nanomolar, greater than about 140 nanomolar, greater than about 160 nanomolar, greater than about 180 nanomolar, greater than about 200 nanomolar, etc. By increasing the subject's homeostatic plasma level of reduced folate to a level that is higher than has been considered in the art to be sufficient, the method of the present invention can be used to protect the subject from unanticipated exposures to ultraviolet radiation.

As discussed above, the present invention involves administering at least one reduced folate to the subject. Suitable reduced folates include all of those discussed above in the context of protecting subjects from ionizing radiation, as well as mixtures of two or more reduced folates; polyglutamyl derivatives; and monoalkyl, dialkyl, monobenzyl, and/or dibenzyl esters of the reduced folate's glutamate side chain. The reduced folates can be in either in the form of a free acid or in the form of a salt, and "reduced folate", as used herein, is also meant to encompass both the free acid and salt forms. Examples of suitable salt forms include hydrochloride, sodium, potassium, calcium, and magnesium salts. The salt form and crystal structure of the reduced folate somewhat affects the reduced folate's stability and solubility, and this can be optimized depending on the needs for a particular formulation. Suitable salt forms also include those in which the counter ion is an organic amine base. The pH of the final composition can also be optimized according to the stability properties of the particular reduced folate used and the other components present in the formulation (if any), as is well understood in the arts of nutrient processing and folate compounds.

As indicated above, the protection of a subject from harmful effects of ultraviolet radiation is carried out with a composition that is substantially free from vitamin B12. As used in this context, "substantially free from vitamin B12" is meant to refer to compositions in which the level of vitamin B12 present in the composition is insufficient to have an appreciable effect on the protection from harmful effects of ultraviolet radiation that the composition affords. Illustratively, in the context of compositions for protecting a subject from harmful effects of ultraviolet radiation, compositions containing no vitamin B12; containing vitamin B12 in an amount that is equal to or less than the recommended daily allowance of vitamin B12 for the subject. As discussed above, "recommended daily allowance", as used herein, is meant to refer to the recommended daily allowance in the United States, which, for vitamin B12, is 6 :g/day.

The reduced folate can be administered alone or in a composition containing, in addition to the reduced folate, one or more other components. Examples of suitable dosage forms include enteral (e.g., oral, intragastric, or transpyloric), parenteral (intramuscular, intravenous, rectal, vaginal, and subcutaneous), topical, and ocular dosage forms.

Illustratively, the reduced folate can be administered orally in the form of a supplement. For example, pills, tablets, chewable tablets, capsules, powders, syrups, suspensions, solutions, and soft chews are suitable forms for administration of reduced folates for protection against ultraviolet radiation. Time delay, slow-release, and enterically-protected formulations can also be used. Suitable dosage forms for orally administered supplements include tablets, dispersible powders, granules, capsules, suspensions, syrups, and elixirs. Inert diluents and carriers for tablets include, for example, calcium carbonate, sodium carbonate, lactose, and talc. Tablets may also contain granulating and disintegrating agents, such as starch and alginic acid; binding agents, such as starch, gelatin, and acacia; and lubricating agents, such as magnesium stearate, stearic acid, and talc. Tablets may be uncoated or may be coated by known techniques to delay disintegration and absorption. Inert diluents and carriers which may be used in capsules include, for example, calcium carbonate, calcium phosphate, and kaolin. Suspensions, syrups, and elixirs may contain conventional excipients, for example, methyl cellulose, tragacanth, sodium alginate; wetting agents, such as lecithin and polyoxyethylene stearate; and preservatives, such as ethyl-p-hydroxybenzoate. Other inert ingredients can also be present in the dosage forms for oral administration.

As discussed above, dosage forms for oral administration can include inert materials, such as fillers, binding agents, stabilizers, sweeteners, including nutritive sweeteners (e.g. sucrose, sorbitol, and other polyols) and non-nutritive sweeteners (e.g. saccharin, aspartame, and acesulfame K), colorants, flavors, buffers, salts, coatings, and the like that are known to those skilled in the art of supplement and pharmaceutical formulation. Additionally or alternatively, the oral dosage forms can also include one or more additional (i.e., in addition to the reduced folate) biologically active materials. Examples of such additional biologically active materials that can be present in the composition include: other vitamins and/or nutrients (e.g., folic acid; vitamin B1; vitamin B2; vitamin B3; vitamin B5; vitamin B6; vitamin C; vitamin A and its precursors, such as beta-carotene; vitamin D; vitamin E including vitamin E isomeric forms and derivatives; vitamin K; biotin; pantothenic acid; methionine; choline; taurine; carnitine; acetyl-carnitine; sugars; lipids; amino acids, such as glutamine, arginine, and methionine; and proteins), reducing agents and antioxidants, thiols (e.g., glutathione and glutathione elevating precursors, glutamine, cysteine, N-acetyl-cysteine, alpha-lipoic acid, cystinyl-glycine, cyctamine, S-allyl cysteine sulfoxide, aminoethylisothiourea, mercaptoethyl guanidine, 2-mercaptopropionylglycine), selenium salts, selenized yeast, selenomethioine, Co-enzyme Q10, amifostine, N-t-butyl hydroxylamine and other N-hydroxylamine derivatives, melatonin, superoxide dismutase, its derivatives and mimetic-metal complexes, chelating agents, phytochemicals, polyphenols, steroids and steroid derivatives (especially those know to be useful for enhancing the protective response of the immune system, for example, DHEA, 5-androstenediol, androstenediol and androstenetriol and their derivatives), extracts of natural products including herbs, chinese herbs, ayurvedic preparations, tea extracts, dithiolthiones, cruciferous vegetables, flavanoids, curcumin, methylxanthines, Gingko biloba extracts, and minerals (e.g., boron, calcium, phosphorus, chromium, copper, manganese, magnesium, nickel, sodium, molybdenum, potassium, iron, selenium, silicon, vanadium, and zinc). As further illustration, the additional biologically active materials that can be used in the ultraviolet protection methods of the present invention include essential nutrients, such as those that have been compiled in a number of published sources, including Modern Nutrition in Health and Disease, 8th ed., Shils et al., eds., Philadelphia:Lea and Febiger (1994).

The reduced folate can also be administered orally as a food that is fortified with one or more reduced folates. Foods can be single-component foods, for example, fruits and fruit juices (e.g., orange juice), dairy products (e.g., milk), vegetables (e.g., spinach), other such single-component foods. Foods can also be multi-component preparations made from two or more single-component foods. Typically, foods contain various concentrations of endogenous reduced folates. Depending on the nature of the processing needed, the fortification is often optimally performed after any especially destructive processing steps, as is well known in the art of food fortification. Since the amount endogenous reduced folates present in the food can vary, it can be advantageous to know the final amount (number of moles) of reduced folate in the food or food preparation, as quantified, for example, by analysis of a sample of a product batch. Many analytical methods (such as microbial growth dependence, folate binding protein based assays, HPLC and GC) are available for measurement of the reduced folate content of foods, food preparations, and supplements.

Irrespective of whether the reduced folate is administered orally to a human subject in the form of a supplement, in the form of a fortified food, or in the form of a food preparation, the total amount of reduced folate administered per dose can be in the range of from about 0.45 micromoles to about 50 micromoles (based on the natural isomer component, if the reduced folate is present as a mixture of isomers), such as from 0.45 micromoles to 50 micromoles, from about 0.9 micromoles to about 50 micromoles, from 0.9 micromoles to 50 micromoles, from about 1.8 micromoles to about 50 micromoles, from 1.8 micromoles to 50 micromoles, from about 0.45 micromoles to about 25 micromoles, from about 0.9 micromoles to about 25 micromoles, from about 1.8 micromoles to about 25 micromoles, from about 0.45 micromoles to about 10 micromoles, from about 0.9 micromoles to about 10 micromoles, from about 1.8 micromoles to about 10 micromoles, from about 0.45 micromoles to about 5 micromoles, from about 0.9 micromoles to about 5 micromoles, from about 1.8 micromoles to about 5 micromoles, from about 0.45 micromoles to about 2 micromoles, from about 0.9 micromoles to about 2 micromoles, and/or from about 1.8 micromoles to about 2 micromoles.

As discussed above, oral administration can be carried out in a single dose, multiple doses, or continuously. The amount of reduced folate contained in a single dose will, of course, depend in part on the dosing regimen and the total amount of reduced folate to be administered to the subject in a given period of time (e.g., per day). Suitable daily dosage ranges for protection against chronic exposure to ultraviolet radiation include: from about 0.45 micromoles to about 15 micromoles, from about 0.9 micromoles to about 15 micromoles, from 0.9 micromoles to 15 micromoles, from about 1 micromoles to about 15 micromoles, from about 2 micromoles to about 12 micromoles, from about 3 micromoles to about 10 micromoles, from about 5 micromoles to about 8 micromoles, etc.

Dosage forms suitable for parenteral administration include solutions, suspensions, dispersions, emulsions, and the like. They may also be manufactured in the form of sterile solid compositions which can be dissolved or suspended in sterile injectable medium immediately before use. They may contain suspending or dispersing agents known in the art. Examples of parenteral administration are intramuscular, intravenous, rectal, and subcutaneous administration.

As mentioned above, the reduced folates can be administered via routes other than oral and parenteral routes. For example, the reduced folates can be administered to the eye in the form of drops, creams, or gel solutions or suspensions adapted for ocular application. The reduced folates can also be administered topically, for example in a conventional topical cream, lotion, spray, or gel matrix. Topical formulations can benefit from incorporation of delivery systems that enhance skin penetration (e.g., liposomes, etc.), as is known in the art. Topical formulations of the reduced folates can also include one or more sunscreen or sunblock agents, such as those known in the art. Illustratively, topical dosage forms can be formulated so as to contain from about 0.05 micromoles to about 1 millimole, such as from 0.05 micromoles to 1 millimole, from about 0.1 micromoles to about 0.5 millimole, from about 0.5 micromoles to about 0.1 millimole (based on the natural isomer component, if the reduced folate is present as a mixture of isomers) per square meter of coverage area. It is preferred that topical formulations be applied, in advance, to the subject's skin in areas of anticipated exposure. Additional applications may be useful to maintain the presence of the reduced folates over a long period of time, or additional applications may be useful in the event of significant exposure to water.

Further details regarding formulating the compositions described hereinabove, such as compositions for topical, enteral, and parenteral administration, can be found, for example, in Handbook of Pharmaceutical Excipients, 3rd Edition (2000), American Pharmaceutical Association; The Theory and Practice of Industrial Pharmacy, 3rd Edition, Lachman et al. 1986; Pharmaceutical Dosage Forms: Tablets Volume Edition, Christopher T, edition, 1995; and Remington's Pharmaceutical Sciences, 2000.

It will be appreciated that the actual preferred amount of reduced folate to be administered according to the present invention will vary according to the particular reduced folate, the particular composition formulated, and the mode of administration. Many factors that may modify the action of the reduced folate (e.g., body weight, sex, diet, time of administration, route of administration, rate of excretion, condition of the subject, drug combinations, reaction sensitivities and severities, and the type, intensity, and duration of the ultraviolet radiation to which the subject is exposed) can be taken into account by those skilled in the art. Administration can be carried out continuously or periodically within the maximum tolerated dose. Optimal administration rates for a given set of conditions can be ascertained by those skilled in the art using conventional dosage administration tests.

The present invention is further illustrated by the following non-limiting examples.

### EXAMPLES

### Example 1 -- Effect of Reduced Folates on X-Ray Destruction of Fluorescein

The prevention of the decay of fluorescein (at nanomolar concentration in 10 mM phosphate buffer pH 7.0) by x-rays was examined in the presence of varied concentrations of 5-methyltetrahydrofolate or 5-formyl-tetrahydrofolate. Plastic vials (1.5 mL) were completely filed with reaction mixture, submersed in a tank of water at a fixed distance from the face of the tank, and exposed to x-ray radiation for 8 minutes to give a total exposure of 25 Gy. Residual fluorescence of the fluorescein was measured in a Perkin Elmer LS 50B fluorometer. A concentration of between about 20 micromolar and 30 micromolar of the above folates provided 50% protection against loss of fluorescence due to destruction of the fluorescein.

### Example 2 -- Effect of Reduced Folates on X-Ray Destruction of DNA

Supercoiled plasmid DNA, PBR 322 was diluted into 10 mM phosphate buffer, pH 7.0 along with varied concentrations of 5-methyltetrahydrofolate or 5-formyl-tetrahydrofolate. Plastic vials (1.5 mL) were completely filed with reaction mixture, submersed in a tank of water at a fixed distance from the face of the tank, and exposed to x-ray radiation for 1.6 minutes to give a total exposure of 5 Gy. The residual supercoiled DNA was separated from the relaxed and linearized forms by elctrophoresis on argarose gels, stained with ethidium bromide, and quantitatively scanned on a Fuji FLA 5000 fluorescence imager. A concentration of between about 30 micromolar and 40 micromolar of the above folates provided 50% protection against loss of supercoiled DNA, i.e. via strand breaks.

### Example 3 -- 5-Methyltetrahydrofolate Inhibits Photosensitization Reactions and Strand Breaks in DNA

In this study, we monitored 5-methyltetrahydrofolate ("5-MTHF") exposed to UVA, UVB, or visible light, in the presence and absence of photosensitizers and tested its effect on the photocleavage of plasmid DNA. 5-MTHF was found to be stable when illuminated alone, yet was depleted in photosensitization reactions while quenching the excited photosensitizer and scavenging singlet oxygen. Even at concentrations below 10 :M, 5-MTHF prevented photodegradation of folic acid and strand-breaks in plasmid DNA in the presence of photoexcited pterin-6 carboxylic acid ("PCA").

In this study, we also reexamined the photochemical properties of folic acid. The pteridine photoproduct of folic acid was originally proposed to be 6-formyl-pterin (6-FP). More recent studies also identified the additional p-aminobenzoylglutamate product and have shown that 6-FP is further degraded to yield PCA. Monitoring the photolysis reactions by HPLC we confirmed that folic acid exposed to UVA initially yields, p-aminobenzoylglutamate and 6-FP, which in turn is oxidized to PCA. Similar results were found with exposure to UVB.

The fate of 5-methyltetrahydrofolate was determined under the same conditions. We found that 5-MTHF exposed to UVA or UVB was degraded only to the same extent observed in the absence of light; e.g. <5% autooxidation in 1 hr. When the photo decay of folic acid was monitored in the presence of 5-MTHF, 5-MTHF inhibited the photolysis of folic acid in a sacrificial way. Folic acid was maintained until 5-MTHF decayed below 1 :M concentration, as shown in Figure 1.

To study the reaction of 5-MTHF in a characterized photosensitization reaction Rose Bengal was used. Since illuminated Rose Bengal can generate superoxide radicals in addition to singlet oxygen (Lee et al., Photochem. Photobiol., 45:79-86 (1987), superoxide dismutase was included in the reaction mixtures. As shown in Figure 2A, 5-MTHF was found to be depleted in the presence of visible-light sensitized Rose Bengal, giving rise to the same products generated by autooxidation. To differentiate the reactions of 5-MTHF with excited state Rose Bengal versus singlet oxygen, the experiments were carried out at various oxygen levels. As further shown in Figure 2A, the rate of depletion of 5-MTHF slowed as the concentration of O₂ increased. However, vigorous sparging of the reaction with argon slowed the depletion to a rate less than that obtained with 100 % oxygen.

To ascertain whether the depletion is mediated at least in part by singlet oxygen, the loss of 5-MTHF was determined under the same experimental conditions with 100 % O₂, but in the presence of increasing concentrations of azide. As shown in Figure 2B, the initial rate for the depletion of 5-MTHF was decreased by about 80% with 5 mM azide indicating the involvement of singlet oxygen. As further shown in Figure 2B, a concentration of 0.5 mM azide decreased the depletion rate of 5-MTHF by about 50 %. In the presence of 50 :M PCA, an efficient generator of singlet oxygen (Thomas et al., Photochem. Photobiol. Sci., 2:245-250 (2003), 5-MTHF at initial concentration of 25 :M was completely depleted by 15 min under UVA exposure.

The rate of loss of 5-MTHF in Rose-Bengal induced-photosensitization reactions in high oxygen levels was found to be decreased in the presence of mM levels of sodium ascorbate , as shown in Figure 2C. The rate of loss of 5-MTHF increased with time in parallel with the loss of the ascorbate. The limitation of the current HPLC method did not allow measurement of the initial rate of loss of 5-MTHF. Under the UVA illumination conditions in the presence of 10 :M PCA, 1 mM ascorbate substantially maintained 25 :M 5-MTHF throughout the reaction.

Although DNA is not a chromophore for UVA radiation, it can be damaged by oxidative reactions initiated by photosensitizers (Fiel et al., Cancer Res., 41:3543-3545 (1981) and Blazek et al., Photochem. Photobiol., 49:607-613 (1989). Exposure of supercoiled plasmid-DNA to UVA for 80 min in the presence of 50 :M folic acid or 50 :M PCA yielded a high percentage of strand-breaks, as shown in Figure 3 (top panel). UV exposure had no damaging effect on the supercoiled plasmid by itself (also shown in the top panel of Figure 3) as previously reported (Hirakawa et al., Arch. Biochem. Biophys., 410:261-268 (2003). Also, folic acid or PCA, at 50 :M, had no effect when incubated with the plasmid for 80 min in the dark.

As noted above, 5-MTHF is depleted when participating in photosensitizing reactions. In order to help maintain its concentration, 0.25 mM 5-MTHF was pumped into reaction mixtures at 1.1 :1/min containing either 50 :M folic acid or 50 :M PCA, and its residual concentration was analyzed by HPLC at various times during UV exposure. In both cases, UVA mediated DNA-damage was inhibited by 5-MTHF which, despite continuous addition, decreased from an initial 10 :M to 0.25 :M by the end of the reaction (Figure 3, bottom panel). Sodium azide at 10 mM also afforded full protection under the same conditions, confirming that the damage is largely mediated by singlet oxygen.

Photodegradation of the natural folate directly by UVA did not occur in the absence of photosensitizers. This can be explained by the lack of significant absorbance of 5-MTHF above 330 nm. Interestingly, the stability of 5-MTHF was also unaffected by UVB irradiation, which overlaps its absorption at 290 nm. It is unlikely that singlet oxygen is produced in the interaction of 5-MTHF with UVB; otherwise 5-MTHF would have been depleted at a faster rate than its autooxidation in the dark. The excited state of 5-MTHF presumably undergoes non-radiative decay and/or releases energy by fluorescence more quickly than its interaction with molecular oxygen.

The slower loss of 5-MTHF in Rose Bengal reaction under high concentrations of O₂ (as shown in Figure 2A) indicates that, in addition to the quenching of the excited state of the photosensitizer, 5-MTHF reacts with singlet oxygen, though at a slower rate. This is illustrated in Figure 4. In a competition reaction, 5-MTHF was found to be about 20 fold more effective than azide in scavenging singlet oxygen in high concentrations of O₂ (Figure 2B). Since the rate constant for the reaction of azide with singlet oxygen in water is 4.5A10⁸ M⁻¹sec⁻¹ (Miskoski et al., Photochem. Photobiol., 57:447-452 (1993), the reaction of 5-MTHF with singlet oxygen appears to be nearly diffusion limited. The lack of full inhibition of 5-MTHF depletion by 5 mM azide may be due to a residual reaction of 5-MTHF with photoactivated Rose Bengal. Saturation of the reaction even with 100% oxygen (i.e. ∼1.4 mM) may have not been sufficient to completely drive the reaction into the singlet oxygen pathway (illustrated in Figure 4).

Generation of strand breaks in DNA has previously been reported to be associated with formation of singlet oxygen in photosensitization reactions (Fiel et al., Cancer Res., 41:3543-3545 (1981); Ravanat et al., J. Photochem. Photobiol. B, 63:88-102 (2001); Ito et al., Biol. Chem., 378:1307-1312 (1997); Ravanat et al., J. Biol. Chem., 276:40601-40604 (2001); and Devasagayam et al., Biochemistry, 30:6283-6289 (1991). Singlet oxygen is produced by PCA and 6-FP and has been suggested to participate in the photodecay of folic acid (Thomas et al., Photochem. Photobiol. Sci., 2:245-250 (2003). This may explain the observed acceleration of the photodecay of folic acid. In the present study, we confirmed that PCA serves as a photosensitizer and catalyzes formation of DNA strand-breaks during exposure to UVA, as reported in Hirakawa et al., Arch. Biochem. Biophys., 410:261-268 (2003). In contrast to Hirakawa's proposed mechanism of electron transfer (type I), the findings that azide inhibited DNA damage in photosensitization reactions mediated by PCA imply that singlet oxygen may also be involved in the damaging effect of folic acid and its photoproducts.

There are many endogenous photosensitizers that may lead to photodamage (Fiel et al., Cancer Res., 41:3543-3545 (1981); Fiel et al., Biochem. Biophys. Res. Commun., 107:1067-1074 (1982); and Mahns et al., Free Radical Res., 37:391-397 (2003). Unmetabolized folic acid has been detected in the plasma of individuals consuming greater than 200 :g of folic acid (Kelly et al., Am. J. Clin. Nutr., 65:1790-1795 (1997). Since tissue levels of unmetabolized folic acid in the skin are currently unknown, the contribution of folic acid to photosensitization reactions remains to be established.

Ascorbate at physiological concentrations was found to decrease the photosensitization-mediated depletion of 5-MTHF, as shown in Figure 2C. Since the second order rate constant for the reaction of singlet oxygen with ascorbate in water is 8.3A10⁶ M⁻¹sec⁻¹ (Chou et al., Biochem. Biophys. Res. Commun., 115:932-937 (1983), the pseudo first order rate with 2 mM ascorbate is 1.7A10⁴ sec⁻¹. This is over 10 times slower than the spontaneous decay rate of singlet oxygen (Studer et al., J. Am. Chem. Soc., 111:7643-7644 (1989). Moreover, ascorbate has previously been reported to have no effect on the generation of protein-derived peroxides in viable Rose Bengal-loaded THP-1 cells (Wright et al., Free. Radic. Biol. Med., 34:637-647 (2003). Thus, ascorbate at this concentration does not significantly protect 5-MTHF by directly removing singlet oxygen or by directly quenching Rose Bengal. Ascorbate may restore 5-MTHF by reducing the initial intermediate in its photodecomposition reactions (most likely its radical cation). The results shown in Figure 2c underestimate the ability of ascorbate to initially maintain 5-MTHF, since a significant fraction is rapidly converted to dehydroascorbate by the time of the first HPLC analysis.

The study reported by Branda and Eaton (Branda et al., Science, 201:625-626 (1978), measured the effect of UV exposure and phototherapy on folate concentrations in plasma of psoriasis patients treated with methoxalen and demonstrated a significant photolysis of folate by UV. Based on this study and epidemiological data, a possible causal relationship between neural tube defect and UV exposure has been proposed (Van Rootselaar, Med. Hypotheses, 41:78-82 (1993) and Jablonski, Med. Hypotheses, 52:581-582 (1999). However, the association between in vivo photolysis of folate and clinical folate deficiency has not yet been clearly established. Our results suggest that the photolysis of folate observed in their study was probably mediated by photosensitizers in the plasma rather than by the intrinsic photolability of 5-MTHF. Depending on photosensitizers and possibly ascorbate status in the skin and/or plasma, long exposure to sunlight may affect the folate pool. Preliminary studies on the nature of the decay products of 5-MTHF indicate that they still contain the p-aminobenzoylglutamate side chain. Since earlier studies of folate catabolites in human urine only looked for the presence of p-aminobenzoylglutamate and its N-acetylated form, the loss of 5-MTHF via oxidative or photolytic decay would have been undetected.

In conclusion, the present results show that, unlike folic acid, 5-MTHF is not photolyzed directly by UV and does not induce cleavage of plasmid DNA. 5-MTHF may afford protection to DNA in sensitization reactions, most likely by quenching the excited state of the photo sensitizer and scavenging singlet oxygen (as summarized in Figure 4). Our results suggest that the natural folate, 5-MTHF, within :M concentrations, may protect biomolecules when photosensitization occurs. Moreover, ascorbate may afford a synergistic effect by maintaining the folate pool against photolytic degradation.

Further details regarding the experiments conducted in this Example 3 are set forth in the following Example 4.

### Example 4 -- Experimental Details Regarding Studies Showing that 5-Methyltetrahydrofolate Inhibits Photosensitization Reactions and Strand Breaks in DNA

The following materials and methods were employed. Sodium azide (>99 %) was purchased from Fluka. Folic acid (98% + 8% H₂O), sodium ascorbate, and superoxide dismutase ("SOD") were purchased from Sigma, (St. Louis, Missouri). 5-Methyl-6S-tetrahydrofolic acid calcium salt was obtained from Eprova (Switzerland). Supercoiled plasmid DNA, PBR 322, (4361 base pairs, molecular weight 2.83 x 10⁶ Daltons) was obtained from Fermentas. Pterin-6-carboxylic acid and 6-formyl-pterin were purchased from Schirck's Laboratories (Jona, Switzerland). Rose Bengal (95% sodium salt) was purchased from Aldrich, and BLUEJUICE™ gel loading buffer and SYBR Safe DNA gel stain were purchased from Invitrogen.

UVA and UVB irradiation were carried out under the following conditions. Samples were exposed to UV light at a distance of 30 cm from either a 15 W UVA lamp, Sylvania 350 BL, (lambda max=365 nm, 820 :W/cm2) or 15 W UVB lamp, UVP (lambda max=302 nm, 820 :W/cm2); both purchased from UVP, Inc., USA. The lamps were mounted in a UVP Model XX-15 lamp holder rested on a XX exposure stand.

To give a solution depth of about 3 mm, 0.5 ml reaction volumes were used in clear 24-well plates. To prevent evaporation during the UVA exposure, samples were covered with MICROAMP™ Optical Adhesive Film (ABI) allowing greater than 80 % transmission above 330 nm. UVB reactions were covered with a quartz window. All reactions were performed at ambient temperature in 10 mM potassium phosphate pH 7.41 (measured at 10 mM and 21°C), equilibrated to air, in the presence of 100 :M diethylenetriaminepentaacetic acid in order to prevent the effect of adventitious redox-active metal ions, pre-empt the Fenton reaction and avoid hydroxyl radical-induced damage.

Strand breaks in DNA were detected as follows. Supercoiled plasmid DNA is converted into a nicked circular-form (relaxed) due to single-strand breaks, and subsequently into a linear form due to double-strands breaks. The three forms can be separated by agarose gel electrophoresis. Supercoiled DNA migrates further than the linear form, which in turn migrates further than the relaxed form.

A mixture of 0.1 :g of plasmid DNA, PBR 322, and folic acid, 5-MTHF or PCA in 10 mM potassium phosphate, pH 7.4, was incubated for 80 min under the above UVA-irradiation conditions for a total exposure of 4 J/cm². To partially maintain the concentration of 5-MTHF, a 0.25 mM solution was continuously added to the reaction mixture at 1.1 :1/min using a Harvard syringe pump with magnetic stirring.

A sample of 10 :1 of the reaction mixture was then subjected to agarose gel electrophoresis after addition of 2 :1 of 10X gel loading buffer. Agarose gel for electrophoresis was prepared by dissolving 0.9 % agarose in 45 mM Tris-borate buffer (pH 8.3), containing 1 mM EDTA. Electrophoresis was run at 4 V/cm for 1 h. The gel was incubated with 1 :g/ml SYBR Safe stain, and the DNA bands were scanned using Fuji FLA-5000 phosphor-imaging system.

Reactions with photoexcited pterin-6-carboxylic acid or folic acid were carried out as follows. Samples were exposed in 24-well plates to UVA light in 0.5 ml reaction volumes containing 5-MTHF and folic acid or PCA in the absence or presence of sodium ascorbate in 10 mM potassium phosphate buffer pH 7.42. Reactions were carried out at ambient temperature, in atmospheric oxygen under the above illumination conditions. Samples were taken by syringe and injected directly into the HPLC.

Photochemical reactions with Rose Bengal were carried out as follows. Samples in septum-stoppered glass cuvettes were illuminated at a distance of 12 cm by light from a 40 W tungsten lamp passed through a Wratten #16 gelatin filter. Reactions were equilibrated with air or sparged with 100 % or 1.8 % O₂ in argon, or extensively with 100 % argon, and illumination was carried out at ambient temperature. Samples were taken through the septum by syringe and injected directly into the HPLC.

5-MTHF photodegradation was assayed by HPLC using the following procedure. Samples were taken during light exposure at various times and analyzed by HPLC on a Luna phenyl-hexyl 5 :m (25 x 0.46 cm) column (Phenomenex) eluted at a flow rate of 1.5 ml/min with ammonium phosphate (20 mM in ammonium), pH 2.8 / acetonitrile (17:1) with detection by UV absorbance using Waters 996, photodiode array spectrometer.

### Example 5 -- Formulation of a Typical Daily Multivitamin Tablet Containing Reduced Folate for Protection Against Chronic Exposures to Ionizing Radiation

A formulation of a typical daily multivitamin tablet containing reduced folate for protection against chronic exposures to ionizing radiation can contain: calcium carbonate; 5-MTHF Ca salt 0.4 to 7 mg (e.g., 4 mg); ascorbic acid 12 to 300 mg (e.g., 60 mg); gelatin; vitamin E acetate 5 to 150 I.U. (e.g., 30 I.U.); starch; niacinamide 4 to 100 mg (e.g., 20 mg); hydroxypropylmethylcellulose; calcium pantothenate 2 to 50 mg (e.g., 10 mg); calcium silicate hydroxypropylcellulose; pyridoxine hydrochloride 0.4 to 10 mg (e.g., 2 mg); riboflavin 0.35 to 8.5 mg (e.g., 1.7 mg); thiamin mononitrate 0.3 to 7.5 mg (e.g., 1.5 mg); beta carotene & vitamin A acetate 1000 to 25000 I.U. (e.g., 5000 I.U.); sodium hexametaphosphate; magnesium stearate; vitamin D 80 to 2000 I.U. (e.g., 400 I.U.); vitamin B12 1 to 30 :g (e.g., 6 :g); and lecithin.

### Example 6 -- Formulation of a Typical Daily Multivitamin Tablet Containing Reduced Folate for Protection Against Chronic Exposures to Ultraviolet Radiation

A formulation of a typical daily multivitamin tablet containing reduced folate for protection against chronic exposures to ultraviolet radiation can contain: calcium carbonate; 5-MTHF Ca salt 0.4 to 7 mg (e.g., 4 mg); ascorbic acid 12 to 300 mg (e.g., 60 mg); gelatin; vitamin E acetate 5 to 150 I.U. (e.g., 30 I.U.); starch; niacinamide 4 to 100 mg (e.g., 20 mg); hydroxypropylmethylcellulose; calcium pantothenate 2 to 50 mg (e.g., 10 mg); calcium silicate hydroxypropylcellulose; pyridoxine hydrochloride 0.4 to 10 mg (e.g., 2 mg); riboflavin 0.35 to 8.5 mg (e.g., 1.7 mg); thiamin mononitrate 0.3 to 7.5 mg (e.g., 1.5 mg); beta carotene & vitamin A acetate 1000 to 25000 I.U. (e.g., 5000 I.U.); sodium hexametaphosphate; magnesium stearate; vitamin D 80 to 2000 I.U. (e.g., 400 I.U.); vitamin B12 1 to 19 :g (e.g., 6 :g); and lecithin.

## Claims

1. A reduced folate for use in a method of protecting a human subject from harmful effects of ionizing radiation, said method comprising administering to the subject an effective amount of at least one said reduced folate; wherein the reduced folate is selected from the group consisting of 5-methyl-tetrahydrofolic acid, and polyglutamyl derivatives thereof.

2. A reduced folate for use according to claim 1, wherein the reduced folate is selected from the group consisting of 5-methyl-(6S)-tetrahydrofolic acid, and polyglutamyl derivatives thereof.

3. A reduced folate for use according to claim 1 wherein the reduced folate is selected from the group consisting of racemic 5-methyl-tetrahydrofolic acid, and polyglutamyl derivatives thereof.

4. A reduced folate according to claim 1 for use according to claim 1, wherein the subject is one who is incurring exposure to a harmful level of ionizing radiation.

5. A reduced folate according to claim 1 for use according to claim 1, wherein the subject is one who is at risk for incurring exposure to a harmful level ionizing radiation.

6. A reduced folate according to claim 1 for use according to claim 1, wherein the ionizing radiation is gamma radiation.

7. A reduced folate according to claim 1 for use according to claim 1, wherein the ionizing radiation is x-ray radiation.

8. A reduced folate according to claim 1 for use according to claim 1, wherein the ionizing radiation is cosmic radiation.

9. A reduced folate according to claim 1 for use according to claim 1, wherein the reduced folate is for oral administration.

10. A reduced folate according to claim 1 for use according to claim 1, wherein the reduced folate is for oral administration in time-release formulation.

11. A reduced folate according to claim 1 for use according to claim 1 wherein the reduced folate is for administration topically.

12. A reduced folate according to claim 1 for use according to claim 1, wherein the reduced folate is for administration intravenously.

13. A reduced folate according to claim 1 for use according to claim 1, wherein the reduced folate is for administration in a composition that comprises the reduced folate.

14. A reduced folate according to claim 13 for use according to claim 13, wherein the composition further comprises one or more additional biologically active materials.

15. A reduced folate according to claim 14 for use according to claim 14, wherein the one or more additional biologically active materials comprises a radioprotective agent.

16. A reduced folate according to claim 15 for use according to claim 15, wherein the radioprotective agent is an iodide salt.

17. A reduced folate according to claim 16 for use according to claim 16, wherein the iodide salt is potassium iodide.

18. A reduced folate according to claim 15 for use according to claim 15, wherein the radioprotective agent is a steroidal radioprotective agent.

19. A reduced folate according to claim 18 for use according to claim 18, wherein the steroidal radioprotective agent is an androstenediol.

20. A reduced folate according to claim 13 for use according to claim 13, wherein the composition further includes, in addition to the reduced folate, one or more other vitamins.

21. A reduced folate according to claim 1 for use according to claim 1, wherein the reduced folate is administered under conditions effective to establish and/or maintain a homeostatic plasma level of reduced folate greater than 20 nanomolar.

22. A reduced folate according to claim 1 for use according to claim 1, wherein the reduced folate is for administration under conditions effective to establish and/or maintain a homeostatic plasma level of reduced folate greater than about 30 nanomolar.

23. A reduced folate according to claim 1 for use according to claim 1, wherein the reduced folate is for administration at a daily dose of from about 0.45 micromoles to about 15 micromoles.

24. A reduced folate according to claim 1 for use according to claim 1, wherein the reduced folate is for administration in a dose containing from about 0.45 micromoles to about 2 millimoles.

25. A radioprotective composition for use in providing radioprotection against ionising radiation to a human subject, the composition comprising: a first radioprotective agent, said first radioprotective agent being a reduced folate; and a second radioprotective agent, wherein the reduced folate is selected from the group consisting of 5-methyl-tetrahydrofolic acid, and polyglutamyl derivatives thereof; wherein the second radioprotective agent is selected from an iodide salt and a steroidal radioprotective agent.

26. A radioprotective composition according to claim 25 for use according to claim 25, wherein the reduced folate is selected from the group consisting of 5-methyl-(6S)-tetrahydrofolic acid, and polyglutamyl derivatives thereof.

27. A radioprotective composition according to claim 25 for use according to claim 25, wherein the reduced folate is selected from the group consisting of racemic 5-methyl-tetrahydrofolic acid, and polyglutamyl derivatives thereof.

28. A radioprotective composition according to claim 25 for use according to claim 25, wherein the second radioprotective agent is an iodide salt.

29. A radioprotective composition according to claim 28 for use according to claim 28, wherein the iodide salt is potassium iodide.

30. A radioprotective composition according to claim 25 for use in providing radioprotection against ionising radiation, the composition comprising a first radioprotective agent and a second radioprotective agent, wherein said first radioprotective agent is a reduced folate selected from the group consisting of 5-methyl - tetrahydrofolic acid, and polyglutamyl derivatives thereof, and the second radioprotective agent is a steroidal radioprotective agent.

31. A radioprotective composition according to claim 30, wherein the steroidal radioprotective agent is an androstenediol.

32. A radioprotective composition according to claim 25 for use according to claim 25, wherein the composition contains no vitamin B12 or contains an amount of vitamin B12 that is equal to or less than 300% of the recommended daily allowance of vitamin B12 for a human subject and said recommended daily allowance is 6 µg/day.

33. A radioprotective composition according to claim 25 for use according to claim 25, wherein the composition comprises from about 0.45 micromoles to about 2 millimoles of reduced folate.

34. A radioprotective composition according to claim 25 for use in providing radioprotection against ionising radiation, the composition comprising a first radioprotective agent and a second radioprotective agent, wherein said first radioprotective agent is a reduced folate selected from the group consisting of 5-methyl - tetrahydrofolic acid, and polyglutamyl derivatives thereof and the second radioprotective agent is potassium iodide and wherein the composition comprises from about 1 milligram to about 500 milligrams of potassium iodide.

35. A radioprotective composition according to claim 34, wherein the composition comprises from about 0.45 micromoles to about 2 millimoles of reduced folate.

36. A reduced folate for use in a method for protecting a human subject from harmful effects of ultraviolet radiation, said method comprising: administering to the subject a composition comprising an effective amount of at least one said reduced folate, wherein said composition is substantially free from vitamin B12 in that it contains no vitamin B12 or contains an amount of vitamin B12 that is equal to or less than the recommended daily allowance of vitamin B12 for a human subject and said recommended daily allowance is 6 µg/day, wherein the reduced folate is selected from the group consisting of 5-methyl-tetrahydrofolic acid, and polyglutamyl derivatives thereof, and monoalkyl, dialkyl, monobenzyl and/or dibenzyl esters of a glutamate side chain of the reduced folate.

37. A reduced folate according to claim 36 for use according to claim 36, wherein the reduced folate is selected from the group consisting of 5-methyl-(6S)-tetrahydrofolic acid, and polyglutamyl derivatives thereof.

38. A reduced folate according to claim 36 for use according to claim 36, wherein the reduced folate is selected from the group consisting of racemic 5-methyl-tetrahydrofolic acid, and polyglutamyl derivatives thereof.

39. A reduced folate according to claim 36 for use according to claim 36, wherein said composition comprises no vitamin B12.

40. A reduced folate according to claim 36 for use according to claim 36, wherein said composition comprises vitamin B12 in an amount that is equal to or less than the recommended daily allowance of vitamin B12 for the subject.

41. A reduced folate according to claim 36 for use according to claim 36, wherein the composition is for administration orally.

42. A reduced folate according to claim 36 for use according to claim 36, wherein the composition is for administration topically.

43. A reduced folate according to claim 36 for use according to claim 36, wherein the composition is a time-release composition and wherein the composition is for administration orally.

44. A reduced folate according to claim 1 for use according to claim 1 wherein the reduced folate is 5-methyl-tetrahydrofolic acid.

45. A reduced folate according to claim 1 for use according to claim 1 wherein the reduced folate is for administration prior to and/or during the subject's exposure to ionizing radiation.

46. A reduced folate according to claim 1 for use according to claim 1 wherein the reduced folate is for administration to the subject at about Tmax prior to the subject being exposed to the ionising radiation.

47. A reduced folate according to claim 1 for use according to claim 1 wherein the reduced folate is for administration in the form of a solution between 0.5 and 2.0 hours prior to the subject being exposed to the ionising radiation.

## Patentansprüche

1. Reduziertes Folat zur Verwendung in einem Verfahren zum Schutz eines menschlichen Patienten vor schädlichen Auswirkungen von ionisierender Strahlung, wobei das Verfahren Folgendes umfasst: die Verabreichung einer wirksamen Menge von mindestens einem reduzierten Folat an den Patienten; wobei das reduzierte Folat aus der Gruppe ausgewählt ist, die aus 5-Methyl-tetrahydrofolsäure und Polyglutamylderivaten davon besteht.

2. Reduziertes Folat zur Verwendung nach Anspruch 1, wobei das reduzierte Folat aus der Gruppe ausgewählt ist, die aus 5-Methyl-(6S)-tetrahydrofolsäure und Polyglutamylderivaten davon besteht.

3. Reduziertes Folat zur Verwendung nach Anspruch 1, wobei das reduzierte Folat aus der Gruppe ausgewählt ist, die aus racemischer 5-Methyl-tetrahydrofolsäure und Polyglutamylderivaten davon besteht.

4. Reduziertes Folat nach Anspruch 1 zur Verwendung nach Anspruch 1, wobei es sich bei dem Patienten um eine Person handelt, die einer schädlichen Menge ionisierender Strahlung ausgesetzt wird.

5. Reduziertes Folat nach Anspruch 1 zur Verwendung nach Anspruch 1, wobei es sich bei dem Patienten um eine Person handelt, bei der die Gefahr besteht, dass sie einer schädlichen Menge ionisierender Strahlung ausgesetzt wird.

6. Reduziertes Folat nach Anspruch 1 zur Verwendung nach Anspruch 1, wobei es sich bei der ionisierenden Strahlung um Gammastrahlung handelt.

7. Reduziertes Folat nach Anspruch 1 zur Verwendung nach Anspruch 1, wobei es sich bei der ionisierenden Strahlung um Röntgenstrahlung handelt.

8. Reduziertes Folat nach Anspruch 1 zur Verwendung nach Anspruch 1, wobei es sich bei der ionisierenden Strahlung um kosmische Strahlung handelt.

9. Reduziertes Folat nach Anspruch 1 zur Verwendung nach Anspruch 1, wobei das reduzierte Folat zur oralen Verabreichung vorgesehen ist.

10. Reduziertes Folat nach Anspruch 1 zur Verwendung nach Anspruch 1, wobei das reduzierte Folat zur oralen Verabreichung vorgesehen ist und die Rezeptur für eine Freigabe im Verlauf der Zeit sorgt.

11. Reduziertes Folat nach Anspruch 1 zur Verwendung nach Anspruch 1, wobei das reduzierte Folat zur topischen Verabreichung vorgesehen ist.

12. Reduziertes Folat nach Anspruch 1 zur Verwendung nach Anspruch 1, wobei das reduzierte Folat zur intravenösen Verabreichung vorgesehen ist.

13. Reduziertes Folat nach Anspruch 1 zur Verwendung nach Anspruch 1, wobei das reduzierte Folat zur Verabreichung in einer Zusammensetzung vorgesehen ist, die das reduzierte Folat enthält.

14. Reduziertes Folat nach Anspruch 13 zur Verwendung nach Anspruch 13, wobei die Zusammensetzung außerdem ein oder mehrere zusätzliche(s), biologisch aktive(s) Material(ien) umfasst.

15. Reduziertes Folat nach Anspruch 14 zur Verwendung nach Anspruch 14, wobei das eine oder die mehreren zusätzliche(n), biologisch aktive(n) Material(ien) eine radioprotektive Substanz umfasst/-en.

16. Reduziertes Folat nach Anspruch 15 zur Verwendung nach Anspruch 15, wobei es sich bei der radioprotektiven Substanz um ein Iodidsalz handelt.

17. Reduziertes Folat nach Anspruch 16 zur Verwendung nach Anspruch 16, wobei es sich bei dem Iodidsalz um Kaliumjodid handelt.

18. Reduziertes Folat nach Anspruch 15 zur Verwendung nach Anspruch 15, wobei es sich bei der radioprotektiven Substanz um eine steroidale radioprotektive Substanz handelt.

19. Reduziertes Folat nach Anspruch 18 zur Verwendung nach Anspruch 18, wobei es sich bei der steroidalen radioprotektiven Substanz um ein Androstenediol handelt.

20. Reduziertes Folat nach Anspruch 13 zur Verwendung nach Anspruch 13, wobei die Zusammensetzung zusätzlich zum reduzierten Folat außerdem ein oder mehrere andere(s) Vitamin(e) umfasst.

21. Reduziertes Folat nach Anspruch 1 zur Verwendung nach Anspruch 1, wobei das reduzierte Folat unter Bedingungen verabreicht wird, die einen homöostatischen Plasmaspiegel reduzierten Folats von mehr als 20 Nanomol hervorrufen bzw. aufrecht erhalten können.

22. Reduziertes Folat nach Anspruch 1 zur Verwendung nach Anspruch 1, wobei das reduzierte Folat zur Verabreichung unter Bedingungen vorgesehen ist, die einen homöostatischen Plasmaspiegel reduzierten Folats von mehr als ungefähr 30 Nanomol hervorrufen bzw. aufrecht erhalten können.

23. Reduziertes Folat nach Anspruch 1 zur Verwendung nach Anspruch 1, wobei das reduzierte Folat für eine Verabreichung in einer täglichen Dosis von ungefähr 0,45 Mikromol bis ungefähr 15 Mikromol vorgesehen ist.

24. Reduziertes Folat nach Anspruch 1 zur Verwendung nach Anspruch 1, wobei das reduzierte Folat für eine Verabreichung in einer Dosis vorgesehen ist, die zwischen ungefähr 0,45 Mikromol und ungefähr 2 Millimol enthält.

25. Radioprotektive Zusammensetzung zur Verwendung als Strahlenschutz gegen ionisierende Strahlung bei einem menschlichen Patienten, wobei die Zusammensetzung das Folgende umfasst: eine erste radioprotektive Substanz, wobei es sich bei der besagten ersten radioprotektiven Substanz um reduziertes Folat handelt; und eine zweite radioprotektive Substanz, wobei das reduzierte Folat aus der Gruppe ausgewählt ist, die aus 5-Methyl-tetrahydrofolsäure und Polyglutamylderivaten davon besteht; wobei die zweite radioprotektive Substanz ausgewählt ist aus einem Iodidsalz und einer steroidalen radioprotektiven Substanz.

26. Radioprotektive Zusammensetzung nach Anspruch 25 zur Verwendung nach Anspruch 25, wobei das reduzierte Folat aus der Gruppe ausgewählt ist, die aus 5-Methyl-(6S)-tetrahydrofolsäure und Polyglutamylderivaten davon besteht.

27. Radioprotektive Zusammensetzung nach Anspruch 25 zur Verwendung nach Anspruch 25, wobei das reduzierte Folat aus der Gruppe ausgewählt ist, die aus racemischer 5-Methyl-tetrahydrofolsäure und Polyglutamylderivaten davon besteht.

28. Radioprotektive Zusammensetzung nach Anspruch 25 zur Verwendung nach Anspruch 25, wobei es sich bei der zweiten radioprotektiven Substanz um ein Iodidsalz handelt.

29. Radioprotektive Zusammensetzung nach Anspruch 28 zur Verwendung nach Anspruch 28, wobei es sich bei dem Iodidsalz um Kaliumjodid handelt.

30. Radioprotektive Zusammensetzung nach Anspruch 25 zur Verwendung als Strahlenschutz gegen ionisierende Strahlung, wobei die Zusammensetzung eine erste radioprotektive Substanz und eine zweite radioprotektive Substanz umfasst, wobei es sich bei der ersten radioprotektiven Substanz um ein reduziertes Folat handelt, ausgewählt aus der Gruppe bestehend aus 5-Methyl-tetrahydrofolsäure und Polyglutamylderivaten davon, und bei der zweiten radioaktiven Substanz um eine steroidale radioprotektive Substanz.

31. Radioprotektive Zusammensetzung nach Anspruch 30, wobei es sich bei der steroidalen radioprotektiven Substanz um ein Androstenediol handelt.

32. Radioprotektive Zusammensetzung nach Anspruch 25 zur Verwendung nach Anspruch 25, wobei die Zusammensetzung kein Vitamin B12 enthält oder eine Menge an Vitamin B12 enthält, die kleiner/gleich 300% der empfohlenen Tagesdosis an Vitamin B12 für einen menschlichen Patienten ist, und die besagte empfohlene Tagesdosis 6 µg/Tag beträgt.

33. Radioprotektive Zusammensetzung nach Anspruch 25 zur Verwendung nach Anspruch 25, wobei die Zusammensetzung zwischen ungefähr 0,45 Mikromol und ungefähr 2 Millimol des reduzierten Folats enthält.

34. Radioprotektive Zusammensetzung nach Anspruch 25 zur Verwendung als Strahlenschutz gegen ionisierende Strahlung, wobei die Zusammensetzung eine erste radioprotektive Substanz und eine zweite radioprotektive Substanz umfasst, wobei es sich bei der ersten radioprotektiven Substanz um ein reduziertes Folat handelt, ausgewählt aus der Gruppe bestehend aus 5-Methyl-tetrahydrofolsäure und Polyglutamylderivaten davon, und bei der zweiten radioaktiven Substanz um Kaliumjodid, und wobei die Zusammensetzung zwischen ungefähr 1 Milligramm und ungefähr 500 Milligramm Kaliumjodid enthält.

35. Radioprotektive Zusammensetzung nach Anspruch 34, wobei die Zusammensetzung zwischen ungefähr 0,45 Mikromol und ungefähr 2 Millimol des reduzierten Folats enthält.

36. Reduziertes Folat zur Verwendung in einem Verfahren zum Schutz eines menschlichen Patienten vor schädlichen Auswirkungen von ultravioletter Strahlung, wobei das besagte Verfahren Folgendes umfasst: Verabreichung einer Zusammensetzung an den Patienten, die eine wirksame Menge mindestens eines besagten reduzierten Folats enthält, wobei die besagte Zusammensetzung im Wesentlichen frei von Vitamin B12 ist, d.h. kein Vitamin B12 enthält oder eine Menge Vitamin B12 enthält, die kleiner/gleich der empfohlenen Tagesdosis Vitamin B12 für einen menschlichen Patienten ist, und die besagte empfohlene Tagesdosis 6 µg/Tag beträgt, wobei das reduzierte Folat ausgewählt ist aus der Gruppe, die besteht aus 5-Methyl-tetrahydrofolsäure und Polyglutamylderivaten davon; und Monoalkyl-, Dialkyl-, Monobenzyl- bzw. Dibenzylester einer Glutamat-Seitenkette des reduzierten Folats.

37. Reduziertes Folat nach Anspruch 36 zur Verwendung nach Anspruch 36, wobei das reduzierte Folat aus der Gruppe ausgewählt ist, die aus 5-Methyl-(6S)-tetrahydrofolsäure und Polyglutamylderivaten davon besteht.

38. Reduziertes Folat nach Anspruch 36 zur Verwendung nach Anspruch 36, wobei das reduzierte Folat aus der Gruppe ausgewählt ist, die aus racemischer 5-Methyl-tetrahydrofolsäure und Polyglutamylderivaten davon besteht.

39. Reduziertes Folat nach Anspruch 36 zur Verwendung nach Anspruch 36, wobei die besagte Zusammensetzung kein Vitamin B12 enthält.

40. Reduziertes Folat nach Anspruch 36 zur Verwendung nach Anspruch 36, wobei die besagte Zusammensetzung eine Menge Vitamin B12 enthält, die kleiner/gleich der empfohlenen Tagesdosis an Vitamin B12 für den Patienten ist.

41. Reduziertes Folat nach Anspruch 36 zur Verwendung nach Anspruch 36, wobei die Zusammensetzung zur oralen Verabreichung vorgesehen ist.

42. Reduziertes Folat nach Anspruch 36 zur Verwendung nach Anspruch 36, wobei die Zusammensetzung zur topischen Verabreichung vorgesehen ist.

43. Reduziertes Folat nach Anspruch 36 zur Verwendung nach Anspruch 36, wobei es sich bei der Zusammensetzung um eine Zusammensetzung mit Freisetzung über einen bestimmten Zeitraum handelt, und wobei die Zusammensetzung zur oralen Verabreichung vorgesehen ist.

44. Reduziertes Folat nach Anspruch 1 zur Verwendung nach Anspruch 1, wobei es sich bei dem reduzierten Folat um 5-Methyl-tetrahydrofolsäure handelt.

45. Reduziertes Folat nach Anspruch 1 zur Verwendung nach Anspruch 1, wobei vorgesehen ist, dass das reduzierte Folat verabreicht wird, bevor bzw. während der Patient der ionisierenden Strahlung ausgesetzt ist.

46. Reduziertes Folat nach Anspruch 1 zur Verwendung nach Anspruch 1, wobei das reduzierte Folat dem Patienten ungefähr Tmax vor Aussetzung gegenüber der ionisierenden Strahlung verabreicht werden soll.

47. Reduziertes Folat nach Anspruch 1 zur Verwendung nach Anspruch 1, wobei das reduzierte Folat zur Verabreichung in Form einer Lösung zwischen 0,5 und 2,0 Stunden vor Aussetzung des Patienten gegenüber der ionisierenden Strahlung vorgesehen ist.

## Revendications

1. Folate réduit destiné à être utilisé dans un procédé de protection d'un sujet humain contre les effets nocifs du rayonnement ionisant, ledit procédé comprenant l'administration au sujet d'une quantité efficace d'au moins un dit folate réduit ; dans lequel le folate réduit est sélectionné dans le groupe constitué par l'acide 5-méthyl-tétrahydrofolique, et des dérivés polyglutamyle de celui-ci.

2. Folate réduit destiné à être utilisé selon la revendication 1, dans lequel le folate réduit est sélectionné parmi le groupe constitué par l'acide 5-méthyl-(6S)-tétrahydrofolique, et des dérivés polyglutamyle de celui-ci.

3. Folate réduit destiné à être utilisé selon la revendication 1 dans lequel le folate réduit est sélectionné parmi le groupe constitué par l'acide 5-méthyl-tétrahydrofolique racémique, et des dérivés polyglutamyle de celui-ci.

4. Folate réduit selon la revendication 1 destiné à être utilisé selon la revendication 1, dans lequel le sujet en est un qui subit une exposition à un niveau nocif de rayonnement ionisant.

5. Folate réduit selon la revendication 1 destiné à être utilisé selon la revendication 1, dans lequel le sujet en est un qui risque de subir une exposition à un niveau nocif de rayonnement ionisant.

6. Folate réduit selon la revendication 1 destiné à être utilisé selon la revendication 1, dans lequel le rayonnement ionisant est un rayonnement gamma.

7. Folate réduit selon la revendication 1 destiné à être utilisé selon la revendication 1, dans lequel le rayonnement ionisant est un rayonnement de rayon x.

8. Folate réduit selon la revendication 1 destiné à être utilisé selon la revendication 1, dans lequel le rayonnement ionisant est un rayonnement cosmique.

9. Folate réduit selon la revendication 1 destiné à être utilisé selon la revendication 1, dans lequel le folate réduit est destiné à une administration par voie orale.

10. Folate réduit selon la revendication 1 destiné à être utilisé selon la revendication 1, dans lequel le folate réduit est destiné à une administration par voie orale dans une formulation à libération prolongée.

11. Folate réduit selon la revendication 1 destiné à être utilisé selon la revendication 1 dans lequel le folate réduit est destiné à une administration par voie topique.

12. Folate réduit selon la revendication 1 destiné à être utilisé selon la revendication 1, dans lequel le folate réduit est destiné à une administration par voie intraveineuse.

13. Folate réduit selon la revendication 1 destiné à être utilisé selon la revendication 1, dans lequel le folate réduit est destiné à une administration dans une composition qui comprend le folate réduit.

14. Folate réduit selon la revendication 13 destiné à être utilisé selon la revendication 13, dans lequel la composition comprend en outre une ou plusieurs matières biologiquement actives supplémentaires.

15. Folate réduit selon la revendication 14 destiné à être utilisé selon la revendication 14, dans lequel les une ou plusieurs matières biologiquement actives supplémentaires comprennent un agent radioprotecteur.

16. Folate réduit selon la revendication 15 destiné à être utilisé selon la revendication 15, dans lequel l'agent radioprotecteur est un sel d'iodure.

17. Folate réduit selon la revendication 16 destiné à être utilisé selon la revendication 16, dans lequel le sel d'iodure est l'iodure de potassium.

18. Folate réduit selon la revendication 15 destiné à être utilisé selon la revendication 15, dans lequel l'agent radioprotecteur est un agent radioprotecteur stéroïdien.

19. Folate réduit selon la revendication 18 destiné à être utilisé selon la revendication 18, dans lequel l'agent radioprotecteur stéroïdien est un androstènediol.

20. Folate réduit selon la revendication 13 destiné à être utilisé selon la revendication 13, dans lequel la composition inclut en outre, en plus du folate réduit, une ou plusieurs autres vitamines.

21. Folate réduit selon la revendication 1 destiné à être utilisé selon la revendication 1, dans lequel le folate réduit est administré dans des conditions efficaces pour établir et/ou maintenir un niveau plasmatique homéostatique de folate réduit supérieur à 20 nanomoles.

22. Folate réduit selon la revendication 1 destiné à être utilisé selon la revendication 1, dans lequel le folate réduit est destiné à une administration dans des conditions efficaces pour établir et/ou maintenir un niveau plasmatique homéostatique de folate réduit supérieur à 30 nanomoles.

23. Folate réduit selon la revendication 1 destiné à être utilisé selon la revendication 1, dans lequel le folate réduit est destiné à une administration en une dose quotidienne d'environ 0,45 micromole à environ 15 micromoles.

24. Folate réduit selon la revendication 1 destiné à être utilisé selon la revendication 1, dans lequel le folate réduit est destiné à une administration en une dose contenant d'environ 0,45 micromole à environ 2 millimoles.

25. Composition radioprotectrice destinée à être utilisée dans la fourniture d'une radioprotection contre le rayonnement ionisant à un sujet humain, la composition comprenant : un premier agent radioprotecteur, ledit premier agent radioprotecteur étant un folate réduit ; et un deuxième agent radioprotecteur, dans laquelle le folate réduit est sélectionné parmi le groupe constitué par l'acide 5-méthyl-tétrahydrofolique, et des dérivés polyglutamyle de celui-ci ; dans laquelle le deuxième agent radioprotecteur est sélectionné parmi un sel d'iodure et un agent radioprotecteur stéroïdien.

26. Composition radioprotectrice selon la revendication 25 destinée à être utilisée selon la revendication 25, dans laquelle le folate réduit est sélectionné parmi le groupe constitué par l'acide 5-méthyl-(6S)-tétrahydrofolique, et des dérivés polyglutamyle de celui-ci.

27. Composition radioprotectrice selon la revendication 25 destinée à être utilisée selon la revendication 25, dans laquelle le folate réduit est sélectionné parmi le groupe constitué par l'acide 5-méthyl-tétrahydrofolique racémique, et des dérivés polyglutamyle de celui-ci.

28. Composition radioprotectrice selon la revendication 25 destinée à être utilisée selon la revendication 25, dans laquelle le deuxième agent radioprotecteur est un sel d'iodure.

29. Composition radioprotectrice selon la revendication 28 destinée à être utilisée selon la revendication 28, dans laquelle le sel d'iodure est l'iodure de potassium.

30. Composition radioprotectrice selon la revendication 25 destinée à être utilisée dans la fourniture d'une radioprotection contre le rayonnement ionisant, la composition comprenant un premier agent radioprotecteur et un deuxième agent radioprotecteur, dans laquelle ledit premier agent radioprotecteur est un folate réduit sélectionné parmi le groupe constitué par l'acide 5-méthyl-tétrahydrofolique, et des dérivés polyglutamyle de celui-ci, et le deuxième agent radioprotecteur est un agent radioprotecteur stéroïdien.

31. Composition radioprotectrice selon la revendication 30, dans laquelle l'agent radioprotecteur stéroïdien est un androstènediol.

32. Composition radioprotectrice selon la revendication 25 destinée à être utilisée selon la revendication 25, dans laquelle la composition ne contient pas de vitamine B12 ou contient une quantité de vitamine B12 qui est inférieure ou égale à 300 % de l'apport journalier recommandé de vitamine B12 pour un sujet humain et ledit apport journalier recommandé est 6 µg/jour.

33. Composition radioprotectrice selon la revendication 25 destinée à être utilisée selon la revendication 25, dans laquelle la composition comprend d'environ 0,45 micromole à environ 2 millimoles de folate réduit.

34. Composition radioprotectrice selon la revendication 25 destinée à être utilisée dans la fourniture d'une radioprotection contre le rayonnement ionisant, la composition comprenant un premier agent radioprotecteur et un deuxième agent radioprotecteur, dans laquelle ledit premier agent radioprotecteur est un folate réduit sélectionné parmi le groupe constitué par l'acide 5-méthyl-tétrahydrofolique, et des dérivés polyglutamyle de celui-ci, et le deuxième agent radioprotecteur est l'iodure de potassium et dans laquelle la composition comprend d'environ 1 milligramme à environ 500 milligrammes d'iodure de potassium.

35. Composition radioprotectrice selon la revendication 34, dans laquelle la composition comprend d'environ 0,45 micromole à environ 2 millimoles de folate réduit.

36. Folate réduit destiné à être utilisé dans un procédé de protection d'un sujet humain contre les effets nocifs du rayonnement ultraviolet, ledit procédé comprenant : l'administration au sujet d'une composition comprenant une quantité efficace d'au moins un dit folate réduit, dans lequel ladite composition est substantiellement exempte de vitamine B12 en ce qu'elle ne contient pas de vitamine B12 ou contient une quantité de vitamine B12 qui est inférieure ou égale à l'apport journalier recommandé de vitamine B12 pour un sujet humain et ledit apport journalier recommandé est 6 µg/jour, dans lequel le folate réduit est sélectionné parmi le groupe constitué par l'acide 5-méthyl-tétrahydrofolique, et des dérivés polyglutamyle de celui-ci, et des esters monoalkyliques, dialkyliques, monobenzyliques et/ou dibenzyliques d'une chaîne latérale glutamate du folate réduit.

37. Folate réduit selon la revendication 36 destiné à être utilisé selon la revendication 36, dans lequel le folate réduit est sélectionné parmi le groupe constitué par l'acide 5-méthyl-(6S)-tétrahydrofolique, et des dérivés polyglutamyle de celui-ci.

38. Folate réduit selon la revendication 36 destiné à être utilisé selon la revendication 36, dans lequel le folate réduit est sélectionné parmi le groupe constitué par l'acide 5-méthyl-tétrahydrofolique racémique, et des dérivés polyglutamyle de celui-ci.

39. Folate réduit selon la revendication 36 destiné à être utilisé selon la revendication 36, dans lequel ladite composition ne comprend pas de vitamine B12.

40. Folate réduit selon la revendication 36 destiné à être utilisé selon la revendication 36, dans lequel ladite composition comprend de la vitamine B12 dans une quantité qui est inférieure ou égale à l'apport journalier recommandé de vitamine B12 pour le sujet.

41. Folate réduit selon la revendication 36 destiné à être utilisé selon la revendication 36, dans lequel la composition est destinée à une administration par voie orale.

42. Folate réduit selon la revendication 36 destiné à être utilisé selon la revendication 36, dans lequel la composition est destinée à une administration par voie topique.

43. Folate réduit selon la revendication 36 destiné à être utilisé selon la revendication 36, dans lequel la composition est une composition à libération prolongée et dans lequel la composition est destinée à une administration par voie orale.

44. Folate réduit selon la revendication 1 destiné à être utilisé selon la revendication 1 dans lequel le folate réduit est l'acide 5- méthyl-tétrahydrofolique.

45. Folate réduit selon la revendication 1 destiné à être utilisé selon la revendication 1 dans lequel le folate réduit est destiné à une administration avant et/ou pendant l'exposition du sujet au rayonnement ionisant.

46. Folate réduit selon la revendication 1 destiné à être utilisé selon la revendication 1 dans lequel le folate réduit est destiné à une administration au sujet à un Tmax environ avant que le sujet ne soit exposé au rayonnement ionisant.

47. Folate réduit selon la revendication 1 destiné à être utilisé selon la revendication 1 dans lequel le folate réduit est destiné à une administration sous la forme d'une solution entre 0,5 et 2,0 heures avant que le sujet ne soit exposé au rayonnement ionisant.
